# EUROPÄISCHE PATENTSCHRIFT

(11) **EP 2 870 230 B1**
(45) Veröffentlichungstag und Bekanntmachung des Hinweises auf die Patenterteilung: **22.08.2018**
(21) Anmeldenummer: 13736765.2
(22) Anmeldetag: 01.07.2013
(51) Int. Cl.: C12M 1/00

(54) **SUBSTRATEINRICHTUNG, KONSERVIERUNGSGERÄT UND VERFAHREN ZUR KRYOKONSERVIERUNG EINER BIOLOGISCHEN PROBE**
SUBSTRATE UNIT, PRESERVATION DEVICE AND METHOD FOR THE CRYOPRESERVATION OF A BIOLOGICAL SAMPLE
SYSTÈME DE SUBSTRAT, APPAREIL DE CRYOCONSERVATION ET PROCÉDÉ DE CRYOCONSERVATION D'UN ÉCHANTILLON BIOLOGIQUE

(30) Priorität: 04.07.2012 DE 102012013267
(43) Veröffentlichungstag der Anmeldung: 13.05.2015
(73) Patentinhaber: Fraunhofer-Gesellschaft zur Förderung der angewandten Forschung e.V., 80686 München (DE)
(72) Erfinder: ZIMMERMANN, Heiko, 97295 Waldbrunn (DE); BEIER, Axel, 88267 Vogt (DE); NEUBAUER, Julia, 66386 St. Ingbert (DE); FUHR, Günter, R., 13187 Berlin (DE)
(74) Vertreter: v. Bezold & Partner Patentanwälte - PartG mbB
(86) Internationale Anmeldenummer: PCT/EP2013/001920
(87) Internationale Veröffentlichungsnummer: WO 2014/005690

(56) Entgegenhaltungen:
- EP-A2- 2 434 873
- WO-A1-96/40858
- WO-A1-99/08513
- WO-A2-2012/024408
- DE-A1- 19 827 875
- DE-T2- 69 633 854
- DE-U1- 8 908 583
- GB-A- 1 539 263
- US-A- 3 499 825
- US-A- 5 257 128
- US-A- 5 964 096
- KUWAYAMA ET AL: "Highly efficient vitrification for cryopreservation of human oocytes and embryos: The Cryotop method", THERIOGENOLOGY, LOS ALTOS, CA, US, Bd. 67, Nr. 1, 6. Dezember 2006 (2006-12-06), Seiten 73-80, XP005794542, ISSN: 0093-691X, DOI: 10.1016/J.THERIOGENOLOGY.2006.09.014

## Beschreibung

Die Erfindung betrifft eine Substrateinrichtung, die zur Kryokonservierung einer biologischen Probe mit biologischen Zellen eingerichtet ist und insbesondere eine Kultivierungsfläche zur Aufnahme der biologischen Probe und eine Kammer zur Aufnahme der Kultivierungsfläche und einer Kultivierungsflüssigkeit umfasst. Des Weiteren betrifft die Erfindung ein Kryokonservierungsgerät, das mit mindestens einer derartigen Substrateinrichtung ausgestattet ist. Des Weiteren betrifft die Erfindung ein Verfahren zur Kryokonservierung einer biologischen Probe mit biologischen Zellen, das mit der genannten Substrateinrichtung oder dem Kryokonservierungsgerät ausgeführt wird. Anwendungen der Erfindung sind bei der Kryokonservierung biologischer Zellen, insbesondere bei der Kryokonservierung von Stammzellen, wie z. B. humanen embryonalen Stammzellen, oder von Keimzellen, wie z. B. Eizellen, gegeben.

Es ist bekannt, für eine Langzeitlagerung humane embryonale Stammzellen (hESC) einer Kryokonservierung (Aufbewahrung im gefrorenen Zustand) zu unterziehen. Beispielsweise ist ein langsames Einfrieren vorgesehen, das auch als "Slow Rate Freezing" bezeichnet wird, wobei erprobte Kryokonservierungsverfahren, welche bei anderen Zelltypen funktionieren, für hESC adaptiert werden. Die Zellen werden von einer Kultivierungsoberfläche abgelöst und in Suspension unter Zusatz von Kryoprotektiva (z. B. 10% DMSO) in Probengefäßen bei geringen Kühlraten eingefroren. Zur Rückgewinnung werden die Proben z. B. in einem Wasserbad aufgetaut (siehe z. B. B.C. Heng et al. in "Biotechnol. Appl. Biochem." Bd. 41, 2005, S. 97-104). Das "Slow Rate Freezing"-Verfahren hat Nachteile in Bezug auf eine geringe Effektivität und Zuverlässigkeit, chemischen und mechanischen Stress beim Ablösen der Zellen von der Kultivierungsoberfläche, eine geringe Überlebensrate der Zellen und eine beschränkte Funktionalität der aufgetauten Zellen. So können die Zellen nach dem Auftauen nur beschränkt auf Kultivierungsoberflächen anwachsen, so dass lange Rekultivierungszeiten erforderlich sind.

Als Alternative zum "Slow Rate Freezing" ist die Kryokonservierung durch Vitrifikation (nahezu verzögerungsfreies Einfrieren oder sprunghaftes Einfrieren) bekannt, wobei die Zellen mit extrem hohen Kühlraten eingefroren werden, um eine Verglasung (bei z. B. - 130°C) zu erzielen. Kühlraten werden z. B. von M. Sansinena et al. in "Cryobiology" Bd. 63(1), 2011, S. 32 ("Numerical simulation of cooling rates in vitrification systems used for oocyte cryopreservation") abgeschätzt. Ein genereller Nachteil der Vitrifikation ist es, dass zur Vermeidung einer Eiskristallbildung hohe Konzentrationen an Kryoprotektiva benötigt werden, die jedoch oft toxische Wirkungen haben und das Kryokonservierungsergebnis beeinträchtigen.

Aufgrund der extrem hohen Abkühlraten ist die herkömmliche Vitrifikation auf kleine Volumina der Proben (Zellen und Kryomedium) beschränkt. Allgemein gilt bisher, dass je geringer das Volumen, je größer das Oberflächen-Volumen-Verhältnis der Probe und je kleiner der Abstand zwischen der Probe und einem Kühlmittel ist, umso größer ist die Wahrscheinlichkeit einer erfolgreichen Vitrifikation.

Eine weitere generelle Beschränkung der herkömmlichen Vitrifikation ergibt sich aus dem Leidenfrost'sche Phänomen, welches sich durch Gasblasenbildung beim Kontakt warmer Oberflächen mit Flüssigstickstoff auszeichnet. Es können isolierende Abschnitte entstehen, welche die Abkühlrate und somit die Chance auf eine erfolgreiche Vitrifikation mindern.

Weiterhin stellt die herkömmliche Vitrifikation hohe Anforderungen an die Materialien, welche hohe und schnelle Temperaturunterschiede tolerieren sollen. Durch Risse oder Verschieben von Bauteilen kann es bei Vitrifikations-Substraten zu Schädigungen an Zellen oder zu einer verminderten Sterilität der Proben kommen. Es besteht daher ein Interesse, geeignete Materialien oder Methoden zu finden, durch die eine Materialschädigung verhindert und Verschleiß minimiert wird.

Bekannt ist z. B. die Vitrifikation im Röhrchen (so genanntes "Straw"), wobei die Zellen von der Kultivierungsoberfläche abgelöst und nach Inkubation in einem Kryomedium in ein offenes oder geschlossenes Straw, ggf. an der Spitze eines Kunststoffstiftes, überführt werden (G. Vajta et al. in "Mol. Reprod. Dev." Bd. 51, 1998, S. 53-58; M. Richards et al. in "Stem Cells" Bd. 22, 2004, S. 779-789; M. Kuwayama et al. in "Theriogenology" Bd. 67, 2007, S. 73-80; und M. Kuwayama et al. in "Reprod. Biomed. Online" Bd. 11, 2005, S. 608-614).

Die Vitrifikation im Straw zeigt zwar gute Überlebensraten. Sie ist jedoch für große Mengen nicht geeignet. Ein sehr geringes Probenvolumen und ein maximiertes Oberflächen-Volumen-Verhältnis schränken die Menge an gleichzeitig vitrifizierbaren Zellen stark ein. Beim Straw ist die Dicke stark limitiert, da bei zu großem Durchmesser das Oberflächen-Volumen-Verhältnis ungünstig für eine erfolgreiche Vitrifikation werden würde. Eine Verlängerung des Straw würde zwar das Oberflächen-Volumen-Verhältnis erhalten, jedoch durch die Handhabung zu sehr langen Inkubationszeiten der Proben und somit zu Zellschädigungen führen.

Des Weiteren ist die Vitrifikation im Straw sehr aufwändig und ein Erfolg stark von der jeweiligen Expertise der durchführenden Person abhängig. Nachteile dieses Verfahrens ergeben sich insbesondere aus der schwierigen Handhabbarkeit der Proben, wodurch es zu Ungenauigkeiten bei der Einstellung der Inkubationszeit in den hochkonzentrierten, toxischen Kryoprotektiva und zu hohem Zellverlust beim Einfrieren und Auftauen kommen kann. Außerdem ist die Anzahl der Zellen, welche durch diese Verfahren vitrifiziert werden können, stark limitiert.

Bei einem weiteren bekannten Vitrifikationsverfahren wird ein so genanntes "Cryoloop" verwendet. Am Cryoloop wird ein Probentröpfchen mit den Zellen in einem Kunstoff-Ring am Ende eines Stiftes gehalten und in Flüssigstickstoff (- 196°C) getaucht (M. Lane et al. in "Fertil Steril." Bd. 72, 1999, S. 1073-1078). Ein Nachteil dieses Verfahrens ergibt sich aus dem direkten Kontakt der Probe mit dem Stickstoff und der Gefahr einer Kontamination der Zellen mit Verunreinigungen im Stickstoff.

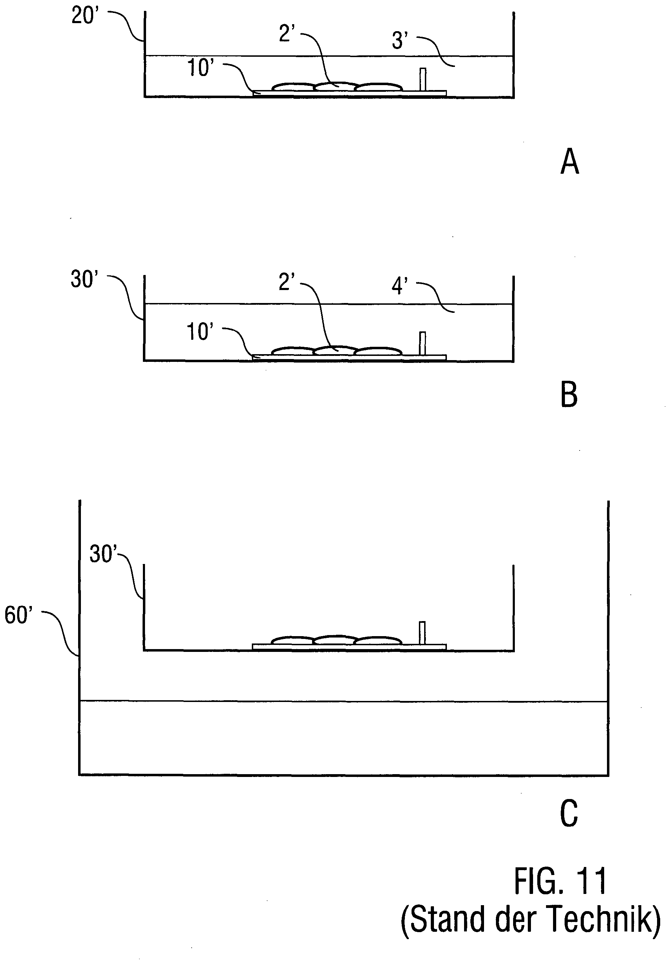

Es ist auch eine adhärente Vitrifikation bekannt, bei der die Zellen im adhärenten Zustand auf einer Kultivierungsfläche vitrifiziert werden (A. F. Beier et al. in "Cryobiology" Bd. 63, 2011, S. 175-185). Das von A. F. Beier vorgeschlagene Verfahren ist schematisch in Figur 11 (Stand der Technik) illustriert. Zunächst werden biologische Zellen 2' auf einer Substratplattform 10' im adhärenten Zustand kultiviert, wobei die Substratplattform 10' in einem Gefäß 20' mit einer Kultivierungsflüssigkeit 3' angeordnet ist (Figur 11A). Die Kultivierungsflüssigkeit 3' umfasst z. B. ein Nährmedium und mindestens ein Kryoprotektivum. Zur Vitrifikation der biologischen Zellen 2' wird die Substratplattform 10' in ein weiteres Gefäß 30' überführt, das als Kühlmedium 4' flüssigen Stickstoff enthält (Figur 11B). Im Gefäß 30' erfolgt die Vitrifikation der Zellen 2'. Für eine dauerhafte Aufbewahrung wird die Substratplattform 10' im Dampf des flüssigen Stickstoffs 4' in einem Stickstofftank 60' angeordnet (Figur 11C).

Auch bei diesem Verfahren ergibt sich ein Nachteil aus dem direkten Kontakt zwischen der Probe und dem Stickstoff und der daraus resultierenden Gefahr einer Kontamination der Zellen mit Verunreinigungen im Stickstoff. Insbesondere eine klinische Anwendung ist nur stark eingeschränkt möglich, da das Risiko mikrobieller Kontaminationen besteht (D. Stoop et al. in "Reprod. Biomed. Online" Bd. 24, 2012 S. 180-185). Zwar gibt es Sterilisierungsmethoden für Flüssigstickstoff, jedoch sind diese zeitaufwändig und kostenintensiv.

Obwohl Flüssigstickstoff direkt nach seiner Herstellung eine hohe Reinheit aufweist und in der Praxis mit pharmazeutischer Qualität kommerziell angeboten wird, können beim Transport und der Lagerung Kontaminationen durch Mikroorganismen und andere Verunreinigungen auftreten. Kontaminationen können durch Aerosol-Bildung an der Oberfläche des Flüssigstickstoffs sogar in die Dampfphase übertragen werden und die Luftqualität beeinträchtigen. Filterverfahren zur Reinigung von Flüssigstickstoff haben sich in der Praxis als umständlich und nicht ausreichend zuverlässig erwiesen.

Es besteht ein Interesse an leicht durchführbaren Vitrifikationsverfahren, insbesondere mit geringeren Anforderungen an die genaue Einhaltung der Inkubationszeit im Kryomedium. Des Weiteren besteht ein Interesse an einer Automatisierbarkeit der Kryokonservierung, um die Nutzung von hESC kostengünstiger, weniger arbeitsintensiv und effizienter zu gestalten. Automatisierte Biobanken beispielsweise erlauben die Lagerung und Nutzung von Stammzellen einer Vielzahl von Patienten oder Organismen.

Für eine erfolgreiche Kryokonservierung ist es ferner wünschenswert, dass die Zellen nach der Vitrifikation, wie z. B. im Straw, und dem Auftauen in der Lage sind, auf einer Kultivierungsoberfläche wieder anzuwachsen, bevor sie z. B. für Experimente oder eine therapeutische Anwendung zur Verfügung stehen. Es besteht ein Interesse, die Zeit zwischen dem Auftauen und der Anwendung zu minimieren, um die Effizienz der Kryokonservierung zu maximieren.

Viele herkömmliche Kultivierungsmethoden erlauben es nicht, die Zellen vor der Kryokonservierung zu vereinzeln oder adhärent zu kultivieren. Explizit sei hier die Kultivierung im hängenden Tropfen ("Hanging Droplet") erwähnt, welche hohe Anforderungen an eine in situ Kryokonservierung stellt. Die Möglichkeit der Anwendbarkeit neuer Techniken zur Kryokonservierung mit diesen Kultivierungstechniken würde große Vorteile mit sich bringen und Spielraum für neue Anwendungen schaffen.

Aus US 5 257 128 ist ein Kryotisch zum Beobachten von Zellen während des Einfrierens und Auftauens in einem kontrollierbaren Flüssigkeitsmilieu und bei einer kontrollierbaren Temperatur im Bereich von 100 °C bis -100 °C bekannt. Der Kryotisch ist jedoch weder für Kultivierungszwecke noch für eine Vitrifikation von Proben geeignet. DE 696 33 854 T2 offenbart ein Verfahren und eine Verpackung zur Erhaltung und Lagerung von kultivierten Gewebeäquivalenten bei tiefen Temperaturen, wobei ein Gefäß, bestehend aus einer Schale, einem Träger mit einer Membran, auf dem das Gewebeäquivalent immobilisiert ist, und einem Deckel verwendet wird. Auch dieses Gefäß ist nicht für Kultivierungszwecke oder eine Vitrifikation von Proben geeignet. Kultivierungsgefäße, die jedoch nicht für Kryokonservierungszwecke ausgelegt sind, werden in US 5 650 325, WO 9 640 858 A1 und GB 1 539 263 A beschrieben.

Das Gebrauchsmuster G 89 08 583 U1 offenbart einen Träger für eine Zellkultivierung, der zwischen zwei Kammern angeordnet ist, die jeweils über Leitungen mit Medien beaufschlagt werden. Aus WO 2010/136818 A2 ist ein Verfahren zur Konservierung von Zellkernen unter Verwendung einer Kryokonservierungs-Vorrichtung bekannt, die mehrere Kammern insbesondere für Präparations- und Konservierungszwecke umfasst.

Die Aufgabe der Erfindung ist es, eine verbesserte Substrateinrichtung und ein verbessertes Verfahren zur Kryokonservierung einer biologischen Probe mit biologischen Zellen bereitzustellen, mit denen Nachteile und Beschränkungen herkömmlicher Techniken zur Kryokonservierung biologischer Proben aufgehoben oder minimiert werden. Die Aufgabe der Erfindung ist es auch, ein verbessertes Kryokonservierungsgerät bereitzustellen, das mit mindestens einer Substrateinrichtung für die Kryokonservierung der biologischen Probe ausgestattet ist. Mit der Erfindung soll insbesondere eine Kryokonservierungstechnik verfügbar gemacht werden, mit der eine vergrößerte Probenmenge gleichzeitig konservierbar ist, die eine reproduzierbare Einstellung von Konservierungsbedingungen ermöglicht, die potentielle Probenkontaminationen ausschließt und/oder eine Vitrifikation biologischer Proben ermöglicht.

Diese Aufgaben werden durch eine Substrateinrichtung, ein Kryokonservierungsgerät bzw. ein Verfahren jeweils mit den Merkmalen der unabhängigen Ansprüche gelöst. Vorteilhafte Ausführungsformen und Anwendungen der Erfindung ergeben sich aus den abhängigen Ansprüchen.

Gemäß einem ersten Gesichtspunkt der Erfindung wird die genannte Aufgabe durch die allgemeine technische Lehre gelöst, eine Substrateinrichtung, insbesondere für die Kryokonservierung einer biologischen Probe, enthaltend biologische Zellen, bereitzustellen, die eine Substratplattform mit einer Kultivierungsfläche und eine erste Kammer umfasst, in der die Kultivierungsfläche der Substratplattform enthalten ist und die zur Aufnahme einer Kultivierungsflüssigkeit eingerichtet ist.

Gemäß der Erfindung ist die Substrateinrichtung mit einer zweiten Kammer ausgestattet, die zur Aufnahme eines Temperierungsmediums (Kühlmedium oder Heizmedium) eingerichtet ist. Gemäß der Erfindung sind die erste Kammer und die zweite Kammer miteinander gekoppelt. Beide Kammern sind aneinander grenzend so verbunden, dass die Substratplattform eine Trennwand zwischen dem Innenraum der ersten Kammer und dem Innenraum der zweiten Kammer bildet. Die erste Kammer (oder: erstes Gefäß, Kultivierungskompartiment) enthält die Kultivierungsfläche mit einer flächenhaften, vorzugsweise ebenen Ausdehnung. Die Kultivierungsfläche ist eine Oberfläche aus einem biologisch verträglichen Material, die zur Aufnahme einer adhärenten Zellkultur oder einer Kultur in hängenden Tropfen geeignet ist. Die zweite Kammer (oder: zweites Gefäß, Stickstoffkompartiment) ist von der ersten Kammer durch die Substratplattform abgegrenzt. Die biologische Probe in der ersten Kammer ist von der Umgebung und insbesondere dem Temperierungsmedium in der zweiten Kammer isoliert. Ein Austausch von Substanzen im flüssigen oder gasförmigen Zustand ist ausgeschlossen.

Vorteilhafterweise erfüllt die erfindungsgemäß zwischen der ersten Kammer und der zweiten Kammer vorgesehene Substratplattform gleichzeitig mehrere Funktionen. Erstens wird an einer Vorderseite der Substratplattform die flächenhafte Kultivierungsfläche bereitgestellt, die eine Aufnahme der biologischen Probe mit einem extrem hohen Oberflächen-Volumen-Verhältnis ermöglicht. Die Größe der Kultivierungsfläche ist frei wählbar, so dass im Vergleich z. B. zur Kryokonservierung in Straws erheblich größere Probenmengen der Kryokonservierung unterzogen werden können.

Zweitens ist die Substratplattform ein festes Bauteil, das sich entlang der flächenhaften Ausdehnung der Kultivierungsfläche erstreckt. Senkrecht zur Kultivierungsfläche, d.h. in Dickenrichtung der Substratplattform, hat diese eine erheblich geringere Ausdehnung, so dass ein in Bezug auf die Wärmeübertragung vom Temperierungsmittel zur biologischen Probe vernachlässigbarer Abstand zwischen der Probe und dem Temperierungsmittel realisiert wird. Die Substratplattform hat die Gestalt einer Platte, Folie oder Materiallage, deren zur ersten Kammer weisende Vorderseite die Kultivierungsfläche bereitstellt und deren entgegengesetzte, zur zweiten Kammer weisende Rückseite einen Abschluss der zweiten Kammer bildet.

Drittens stellt die Substratplattform die Trennung von biologischer Probe und Temperierungsmittel, insbesondere die Trennung von biologischer Probe und flüssigem Stickstoff sicher. Dies ermöglicht im Vergleich zu herkömmlichen Techniken neue Anwendungen der Substrateinrichtung mit erhöhter Zuverlässigkeit, insbesondere medizinische und biotechnologische Anwendungen, ohne dass besondere Vorkehrungen zur Bereinigung des Temperierungsmediums getroffen werden müssen.

Gemäß einem zweiten Gesichtspunkt der Erfindung wird die oben genannte Aufgabe durch die allgemeine technische Lehre gelöst, ein Verfahren zur Kryokonservierung einer biologischen Probe bereitzustellen, bei dem in einem ersten Schritt biologische Zellen an einer Kultivierungsfläche einer Substratplattform in einer ersten Kammer in einer Kultivierungsflüssigkeit angeordnet, insbesondere kultiviert, werden und in einem zweiten Schritt die Temperatur der Substratplattform abgesenkt und die biologische Probe in einen gefrorenen Zustand überführt wird, indem ein Kühlmedium in eine zweite, an die erste Kammer angrenzende Kammer gefüllt wird, wobei die Substratplattform eine Trennwand zwischen der ersten Kammer und der zweiten Kammer bildet. Vorzugsweise wird das Verfahren mit der Substrateinrichtung gemäß dem oben genannten ersten Gesichtspunkt der Erfindung realisiert. Vorzugsweise kann durch das Einfüllen des Kühlmediums in die zweite Kammer die Temperatur der Substratplattform mit der biologischen Probe sprungartig so gesenkt werden, dass eine Vitrifikation der biologischen Probe erzielt wird.

Das erfindungsgemäße Verfahren ist mit verschiedenen Arten von biologischen Proben ausführbar. Der Begriff "biologische Probe" bezeichnet jede Zusammensetzung aus biologischen Zellen und einer Kultivierungsflüssigkeit. Die Kultivierungsflüssigkeit bildet um die Zellen einen Flüssigkeitsfilm oder einen Flüssigkeitstropfen. Die biologischen Zellen umfassen vereinzelte Zellen, Zellgruppen oder Zellkolonien, insbesondere im adhärenten oder im suspendierten Zustand. Die biologische Probe kann Zellen eines einzigen Zelltyps (identische Zellen) oder Zellen verschiedener Zelltypen, wie z. B. Stammzellen und differenzierte Zellen, umfassen. Gemäß einer vorteilhaften Variante der Erfindung können z. B. auf der Kultivierungsfläche Zellen verschiedener Zelltypen im adhärenten Zustand einer gemeinsamen Kultivierung (Kokultivierung) unterzogen werden, bevor das Einfrieren unter der Einwirkung des Kühlmittels erfolgt. Die Kultivierungsflüssigkeit (Kryomedium) umfasst allgemein mindestens ein Nährmedium und mindestens ein Kryoprotektivum, wie z. B. DMSO, Propandiol oder Ethylenglykol. Das Kryoprotektivum kann insbesondere eine Zusammensetzung aus 20% DMSO, 20% Ethylenglykol und 300 mM Trehalose umfassen. Die Bereitstellung der Kultivierungsflüssigkeit kann einmalig oder als Folge verschiedener Kultivierungsflüssigkeiten, die jeweils verschiedene Nährmedien und/oder Kryoprotektiva enthalten, erfolgen.

Ein besonderer Vorteil des erfindungsgemäßen Verfahrens ist es, dass Probleme aufgrund des Leidenfrost'schen Phänomens, die oben beschrieben wurden, mit der erfindungsgemäßen Technik minimiert werden. Eventuell entstehende Gasblasen, die beim Einfüllen des Kühlmediums in die zweite Kammer entstehen, steigen in der zweiten Kammer nach oben auf und bewegen sich dabei von der Substratplattform weg. Die Bildung unerwünschter Abschnitte thermischer Isolation wird dadurch vermieden.

Gemäß einem dritten Gesichtspunkt der Erfindung wird die oben genannte Aufgabe durch die allgemeine technische Lehre gelöst, ein Kryokonservierungsgerät bereitzustellen, das mindestens eine Substrateinrichtung gemäß dem oben genannten ersten Gesichtspunkt der Erfindung und eine Dreheinrichtung umfasst, die zur Aufnahme und zum Drehen (Schwenken) der mindestens einen Substrateinrichtung konfiguriert ist. Die Substrateinrichtung ist im Kryokonservierungsgerät drehbar angeordnet. Gemäß der Erfindung kann die Substrateinrichtung mit der Dreheinrichtung zwischen verschiedenen Zuständen gedreht (verschwenkt) werden, die sich in Bezug auf die Anordnung der ersten Kammer und der zweiten Kammer in vertikaler Richtung, d.h. in Bezug auf die Gravitationsrichtung, unterscheiden. Mit der Dreheinrichtung kann die Substrateinrichtung zwischen einem Kultivierungszustand, in dem die erste Kammer in vertikaler Richtung über der zweiten Kammer angeordnet ist und die Substratplattform einen Boden der ersten Kammer bildet, und einem Temperierungszustand verschwenkt werden, in dem die zweite Kammer über der ersten Kammer angeordnet ist und die Substratplattform einen Boden der zweiten Kammer bildet.

Gemäß einer bevorzugten Anwendung der Erfindung ist die Substrateinrichtung für eine Vitrifikation der biologischen Probe an der Kultivierungsfläche konfiguriert. Hierzu weist die Substratplattform vorzugsweise eine Dicke und eine Wärmeleitfähigkeit auf, die so gewählt sind, dass bei Benetzung der zum Innenraum der zweiten Kammer vorzugsweise freiliegenden Rückseite der Substratplattform mit einem Kühlmedium mit einer Temperatur unterhalb der Glasübergangstemperatur der Probe, z. B. gleich oder unterhalb von - 130 °C, insbesondere mit flüssigem Stickstoff, die Temperatur der biologischen Probe verzögerungsfrei auf die Temperatur des Kühlmediums eingestellt wird. Die Glasübergangstemperatur der Probe liegt z. B. bei - 130 °C, kann jedoch je nach der Konzentration und den Bedingungen, z. B. Druck höher oder niedriger sein. Die Dicke und die Wärmeleitfähigkeit der Substratplattform sind insbesondere so gewählt, dass eine Kühlrate oberhalb von minus 5000°/s, besonders bevorzugt oberhalb von minus 37500°/s erzielt wird. Vorteilhafterweise werden damit praktisch interessierende Kühlraten erzielt (siehe oben, M. Sansinena et al.).

Vorteilhafterweise kann die Dicke der Substratplattform vom Fachmann, insbesondere in Abhängigkeit von der gewünschten Kühlrate, der lateralen Ausdehnung und der erforderlichen mechanischen Stabilität, gewählt werden. Für die Vitrifikation der biologischen Probe hat es sich als vorteilhaft erwiesen, wenn die Substratplattform gemäß einer bevorzugten Variante der Erfindung eine Dicke unterhalb von 200 µm, besonders bevorzugt unterhalb von 120 µm, wie z. B. 100 µm oder darunter aufweist. Des Weiteren kann die Vitrifikation der biologischen Probe vorteilhafterweise gefördert werden, wenn die Substratplattform aus Glas, Kunststoff, Halbleitermaterial, wie z. B. Silizium, oder Metall, wie z. B. Kupfer, Gold oder Silber gebildet ist. Allgemein werden biokompatible Materialien mit einer hohen Wärmeleitfähigkeit, wie z. B. den Wärmeleitfähigkeiten der genannten Materialien, verwendet. Eine Substratplattform aus Glas oder Kunststoff hat Vorteile in Bezug auf eine hohe mechanische Stabilität und die Verfügbarkeit biokompatibler Materialien. Des Weiteren hat eine Substratplattform aus Halbleitermaterial oder Metall ebenfalls Vorteile in Bezug auf eine hohe Stabilität und darüber hinaus in Bezug auf die hohe Wärmeleitfähigkeit. Die Verwendung von Metall für die Substratplattform oder besonders bevorzugt für die gesamte Substrateinrichtung kann des Weiteren aufgrund der Wärmekapazität von Metallen von Vorteil sein. Selbst beim Ausfall einer Kühlung, z. B. in einem Stickstofftank, könnte die erforderliche Konservierungstemperatur der Probe zumindest temporär erhalten bleiben, so dass Probenverluste minimiert werden.

Gemäß einer weiteren vorteilhaften Variante kann die Substratplattform aus einem transparenten Material hergestellt sein. Die Substratplattform kann besonders bevorzugt so gebildet sein, dass die biologische Probe an der Kultivierungsfläche einer optischen, insbesondere mikroskopischen Untersuchung, unterzogen werden kann. Vorteilhafterweise wird damit eine Probenbeobachtung während des Einfrierens und während der Kryokonservierung ermöglicht.

Gemäß einer weiteren vorteilhaften Ausführungsform der Erfindung ist die Substrateinrichtung mit einer Substrathalterung ausgestattet, die für eine flüssigkeitsdichte, lösbare Verbindung der Substratplattform mit dem ersten und/oder der zweiten Kammer konfiguriert ist. Vorteilhafterweise stellt die Substrathalterung eine Verankerung für austauschbare Substratplattformen dar. Die Substrateinrichtung kann mit verschiedenen Kultivierungsflächen ausgestattet werden, die z. B. in Abhängigkeit von den zu konservierenden Zelltypen und/oder der Anwendung der Erfindung gewählt werden können.

Gemäß einer weiteren vorteilhaften Variante der Erfindung ist die erste und/oder die zweite Kammer der Substrateinrichtung mit einem Ausgleichsabschnitt ausgestattet. Der Ausgleichsabschnitt ist zwischen der Substratplattform und den übrigen Teilen der ersten und/oder der zweiten Kammer angeordnet und zur Aufnahme temperaturabhängiger mechanischer Spannungen zwischen der Substratplattform und der ersten Kammer eingerichtet. Wenn die Substratplattform und die übrigen Teile der ersten oder der zweiten Kammer aus verschiedenen Materialien hergestellt sind, können bei der Abkühlung oder der Erwärmung der Substrateinrichtung mechanische Spannungen auftreten, die durch den Ausgleichsabschnitt kompensiert werden. Der Ausgleichsabschnitt ist z. B. eine Dehnungsfuge, die eine flexible Pufferzone zwischen der Substratplattform und deren Halterung in der ersten und/oder in der zweiten Kammer bildet.

Alternativ oder zusätzlich kann die erste Kammer der Substrateinrichtung mit einem Druckausgleichsventil ausgestattet sein. Das Druckausgleichsventil ist eingerichtet, einen eventuellen Überdruck in der ersten Kammer gegenüber der Umgebung auszugleichen. Der Überdruck kann z. B. bei der Erwärmung der Substrateinrichtung bei der Rückgewinnung der biologischen Probe auftreten.

Gemäß einer weiteren Variante der Erfindung kann die Substratplattform ein integraler Bestandteil der ersten Kammer oder der kompletten Substrateinrichtung sein. Beispielsweise kann die Substrateinrichtung mit den ersten und zweiten Kammern und der Substratplattform einstückig gebildet sein. In diesem Fall ergeben sich Vorteile in Bezug auf die mechanische Stabilität und Kompaktheit der Substrateinrichtung. Diese kann z. B. mit einem Spritzgussverfahren aus Kunststoff hergestellt sein.

Die erfindungsgemäße Substrateinrichtung ermöglicht vorteilhafterweise verschiedene Varianten der Zufuhr der Kultivierungsflüssigkeit und/oder eines Temperierungsmediums. Beispielsweise kann eine manuelle Zufuhr vorgesehen sein, bei der die jeweiligen Medien mit einer Gießeinrichtung in die erste oder zweite Kammer eingefüllt werden. Alternativ kann die Substrateinrichtung mit einer Zufuhreinrichtung ausgestattet sein, die für die Zuführung der Kultivierungsflüssigkeit und/oder eines der Temperierungsmedien konfiguriert ist. Diese Variante bietet Vorteile für die automatisierte Anwendung der Substrateinrichtung und die erhöhte Reproduzierbarkeit bei der Zuführung der Medien und der Einhaltung vorgegebener Konservierungsprotokolle. Vorteilhafterweise kann die Zufuhreinrichtung z. B. eine Mikrofluidikeinrichtung umfassen, die in eine Wand oder einen Deckel der ersten Kammer oder der zweiten Kammer integriert ist. Die Mikrofluidikeinrichtung umfasst z. B. einen Fluidikchip, wie er aus der fluidischen Mikrosystemtechnik an sich bekannt ist und der Leitungs- und Dosierelemente zur Medienzufuhr aufweist. Alternativ oder zusätzlich kann die Zufuhreinrichtung mindestens eine Medienleitung umfassen, die in das Innere der ersten Kammer oder der zweiten Kammer mündet. Ebenso wie die Mikrofluidikeinrichtung kann die Medienleitung alternativ in die Substratplattform integriert sein.

Wenn gemäß einer weiteren vorteilhaften Ausführungsform der Erfindung die erste Kammer und die zweite Kammer der Substrateinrichtung lösbar miteinander verbunden sind, können sich weitere Vorteile für die Anpassung der Substrateinrichtung an die Anforderung einer konkreten Anwendung der Erfindung und für die Handhabung der Substrateinrichtung, z. B. bei der Reinigung und bei der Beschickung der ersten Kammer mit der biologischen Probe ergeben. Gemäß einer ersten Variante kann die zweite Kammer mit einem Kammerrahmen fest verbunden sein, die zur lösbaren Aufnahme der ersten Kammer eingerichtet ist. Vorteilhafterweise erfüllt in diesem Fall die zweite Kammer mit dem Kammerrahmen eine Doppelfunktion erstens in Bezug auf die Temperierung der biologischen Probe in der ersten Kammer und zweitens in Bezug auf die Halterung der ersten Kammer. Gemäß einer weiteren Variante können die erste Kammer und die zweite Kammer über eine Schraubverbindung miteinander verbunden sein.

Die Umsetzung der Erfindung ist nicht auf die Kopplung einer einzigen ersten Kammer mit einer einzigen zweiten Kammer beschränkt. Insbesondere für die Konservierung von Zellen verschiedener Zelltypen kann es von Vorteil sein, wenn die erste Kammer in mehrere Teilkammern unterteilt ist, die jeweils zur Aufnahme einer separaten Probe angeordnet sind. Die Teilkammern sind nebeneinander und an die zweite Kammer grenzend angeordnet, wobei die Substratplattform eine gemeinsame Trennwand zwischen den Teilkammern und der zweiten Kammer bildet. Vorteilhafterweise können alle Proben in allen Teilkammern gleichzeitig mit dem Temperierungsmedium in der zweiten Kammer temperiert, z. B. eingefroren oder aufgetaut werden.

Gemäß der Erfindung ist die Substrateinrichtung mit einer Kammerhalterung ausgestattet, die für eine schwenkfähige Aufnahme der Substrateinrichtung in einem Kryokonservierungsgerät, insbesondere in dem Kryokonservierungsgerät gemäß dem dritten Gesichtspunkt der Erfindung konfiguriert ist. Die Kammerhalterung umfasst zwei in einer Ebene parallel zur Ausdehnung der Substratplattform angeordnete Trägerelemente, die mit dem Kryokonservierungsgerät, koppelbar sind. Die Trägerelemente sind z. B. Zapfen, die in Lagern des Trägers sitzen, oder Drehlager zur Aufnahme von Zapfen des Trägers. Vorteilhafterweise ermöglicht die Kammerhalterung, dass die Substrateinrichtung schnell und reproduzierbar um ihre Querachse zwischen dem Kultivierungszustand und dem Konservierungszustand verschwenkt werden kann.

Vorteilhafterweise ist die erfindungsgemäße Kryokonservierung mit verschiedenen Arten von Zellkulturen ausführbar, wobei sich die Zellkulturen in Bezug auf die Bereitstellung der biologischen Zellen an der Kultivierungsfläche unterscheiden. Gemäß einer ersten Variante des erfindungsgemäßen Verfahrens erfolgt eine Kryokonservierung von adhärenten biologischen Zellen, d.h. von biologischen Zellen, die auf der Kultivierungsfläche adhärent (anhaftend) angeordnet sind. In diesem Fall wird in einem ersten Teilschritt die Substrateinrichtung in einem Kultivierungszustand bereitgestellt, in dem die erste Kammer über der zweiten Kammer angeordnet ist und die Substratplattform den Boden der ersten Kammer bildet. Die adhärente Zellkultur auf der Kultivierungsfläche ist mit der Kultivierungsflüssigkeit bedeckt. Die biologischen Zellen werden auf der Kultivierungsfläche einer Kultivierung unterzogen, d.h. einem Zellwachstum und ggf. einer Zellvermehrung unter der Wirkung von Nährmedien und/oder Differenzierungsfaktoren in der Kultivierungsflüssigkeit. In einem weiteren Teilschritt wird die Substrateinrichtung in einen Temperierungszustand verschwenkt, in dem die zweite Kammer über der ersten Kammer angeordnet ist und die Substratplattform einen Boden der zweiten Kammer bildet. Die Kultivierungsflüssigkeit fließt aus der ersten Kammer ab, so dass vorteilhafterweise die adhärent auf der Kultivierungsfläche verbleibenden Zellen nur von einem dünnen Flüssigkeitsfilm bedeckt bleiben, der durch die Oberflächenspannung der verbleibenden Kultivierungsflüssigkeit gebildet wird. Damit wird das Volumen der biologischen Probe minimiert, die der Kryokonservierung unterzogen wird soll. Im Temperierungszustand wird das Kühlmedium, wie z. B. flüssiger Stickstoff, in die zweite Kammer eingefüllt. Das Kühlmedium bedeckt die nach oben weisende Rückseite der Substratplattform, so dass diese gemeinsam mit der auf der Kultivierungsfläche angeordneten biologischen Probe verzögerungsfrei abgekühlt wird.

Gemäß einer zweiten Variante des erfindungsgemäßen Verfahrens wird eine "Hanging-Droplet"-Kultivierung und eine Vitrifikation von Zellen im hängenden Tropfen ermöglicht. Es erfolgt eine Kryokonservierung von biologischen Zellen im nichtadhärenten Zustand. Die biologischen Zellen werden in hängenden Tropfen eingefroren. Hierzu wird in einem ersten Teilschritt die Substrateinrichtung in dem Temperierungszustand bereitgestellt, in dem sich die zweite Kammer über der ersten Kammer befindet und die Substratplattform den Boden der zweiten Kammer bildet. Die Kultivierungsfläche der Substratplattform ist horizontal ausgerichtet, wobei die Flächennormale der Kultivierungsfläche senkrecht nach unten, d.h. in Gravitationsrichtung, weist. An der Kultivierungsfläche werden hängende Tropfen der Kultivierungsflüssigkeit angebracht, in denen biologische Zellen einzeln oder in Zellgruppen platziert werden. Optional kann vor dem Einfrieren eine Kultivierung der biologischen Zellen in den hängenden Tropfen vorgesehen sein. In einem zweiten Teilschritt erfolgt wie bei der ersten Ausführungsform des erfindungsgemäßen Verfahrens die Befüllung der zweiten Kammer mit dem Kühlmedium, so dass die Substratplattform und die biologische Probe sprungartig eingefroren werden.

Die erfindungsgemäße Substrateinrichtung bietet auch Vorteile bei der Rückgewinnung der kryokonservierten Zellen. Die zweite Kammer kann zum Auftauen der biologischen Probe verwendet werden. Hierzu wird ein Heizmedium, wie z. B. Wasser mit einer vorbestimmten Auftautemperatur von z. B. 37 °C in die zweite Kammer gefüllt, wenn die Substrateinrichtung im Temperierungszustand ist. Die Substratplattform mit der biologischen Probe wird durch das Heizmedium erwärmt, bis die biologische Probe aufgetaut ist. Anschließend können die Zellen aus der ersten Kammer entnommen oder in diesem einer weiteren Kultivierung unterzogen werden.

Weitere Vorteile der Erfindung sind im folgenden zusammengefasst. Mit der Erfindung können die Vorteile einer adhärenten Kryokonservierung mit denen der Vitrifikation durch Flüssigstickstoff kombiniert werden. Da die Zellen im adhärenten Zustand kryokonserviert werden können, ist vor der Vitrifikation keine Behandlung der Zellen mit Enzymen, wie etwa Trypsin oder Kollagenase nötig, etwa um die Zellen vom Substrat abzulösen, und nach dem Wiederauftauen müssen die Kolonien nicht erst wieder anwachsen, bevor eine weitere Kultur möglich ist. Zudem bleiben die Zell-Zell-Kontakte in ihrer ursprünglichen Form erhalten und der auftretende Zellstress dadurch noch stärker verringert.

Des Weiteren sind die Überlebensraten, sowie die Funktionalität der Zellen nach der Kryokonservierung denen einer Vitrifikation im Straw überlegen oder mit ihnen vergleichbar. Ein Vorteil gegenüber dem Straw ist jedoch die Möglichkeit, eine große Menge an Zellen gleichzeitig zu konservieren. Beispielsweise kann durch eine Vergrößerung der Kultivierungsoberfläche der Substrateinrichtung leicht eine Vielzahl an Kolonien auf einfache Art vitrifiziert werden. Auch sind die Inkubationszeiten in den Kryoprotektiva ggf. mit hohen Konzentrationen zugeführt werden, genau definierbar, da jede Zellkolonie gleichzeitig mit den jeweiligen Medien in Kontakt kommt und nicht wie beim Straw durch die Bearbeitung jeder Kolonie einzeln je nach Kolonie unterschiedlich.

Ein weiterer Vorteil ist die leichte Automatisierbarkeit des erfindungsgemäßen Verfahrens. Die Proben müssen nicht durch arbeitsaufwändiges, manuelles Pipettieren von einem zum nächsten Kryomedium überführt und anschließend in den Straw gesaugt werden. Stattdessen kann ein Mediumwechsel durch einfaches Absaugen oder sogar durch automatisches Umdrehen des Substrats durchgeführt werden. Die einfache Handhabung des Systems sorgt dafür, dass ein Konservierungserfolg nicht von der jeweiligen Expertise und Geschicklichkeit der durchführenden Person abhängig ist, sondern universell mit ähnlichem Erfolg durchgeführt werden kann.

Der Gefahr einer Kontamination bisheriger adhärenter Vitrifikationsverfahren wird durch das "Zwei-Kammer-System" gemäß der Erfindung minimiert. Da zu jeder Zeit zwischen dem Kultivierungs- und dem Stickstoffkompartiment eine physische Barriere in Form der Kultivierungsoberfläche vorliegt kommt es zu keinem Kontakt zwischen Zellmaterial und potentiell unsterilem Stickstoff. Das Kultivierungskompartiment (erste Kammer) kann zudem durch einen Deckel von der Umgebung getrennt, insbesondere vor Raumluft geschützt sein. Dadurch wird die Anwendung des Systems für therapeutische Zwecke vereinfacht, und es ist nicht nötig, sterilen Stickstoff für die Vitrifikation oder die Lagerung im Tank zu verwenden.

Die physikalischen Rahmenbedingungen für eine erfolgreiche Vitrifikation, wie etwa minimiertes Probenvolumen und maximiertes Oberflächen-Volumen-Verhältnis für eine möglichst hohe Abkühlrate, werden bei der Erfindung durch mehrere Gesichtspunkte begünstigt. Zunächst kommt es durch die adhärente Kultivierung und die Vitrifikation im hängenden Zustand ("über Kopf") zu einer Ausbildung eines minimalen Flüssigkeitsfilmes über den Zellen. Überschüssiges Medium fließt nach unten ab und führt somit zu keiner unerwünschten Vergrößerung des Probenvolumens. Dadurch kann immer genügend Kryomedium für die Inkubation verwendet werden, ohne Gefahr eines zu großen Volumens bei der Vitrifikation. Durch den minimalen Flüssigkeitsfilm kann das toxische Kryomedium nach dem Wiederauftauen auch leicht durch kleine Mengen eines Waschmediums verdünnt werden. Zellschädigungen durch toxische Kryomedien sind somit minimal. Des Weiteren begünstigt die flache Form der angewachsenen Probe, z. B. Zellkolonie, die Abkühlrate positiv. Der Abstand zwischen Kühlmittel und Zellen ist minimal und das Oberflächen-Volumen-Verhältnis der flachen Zellkolonie größer als z. B. im Straw. Zusätzlich können die Zellkolonien in Co-Kultur mit anderen Zelltypen kryokonserviert werden. Somit entfällt nach dem Wiederauftauen ein arbeitsintensives Ansetzen von Co-Kultur, und die Zeit, welche die Zellen außerhalb der Co-Kultur verbringen, wird minimiert. Dieser Vorteil ist besonders bei der Co-Kultur zwischen hESC und Maus-Feeder-Zellen ausgeprägt.

Durch die Möglichkeit der Verwendung unterschiedlicher Kultivierungsoberflächen kann die erfindungsgemäße Substrateinrichtung je nach Anwendung und Zelltyp manipuliert werden. Durch verbesserte Wärmeleitung der Oberfläche kann ein Vitrifikationserfolg weiter maximiert werden. Durch eine spezielle Verankerung für verschiedene Kulturoberflächen können diese je nach Anwendung ausgetauscht werden. Thermisch bedingte Volumenveränderungen der verwendeten Materialen können durch eine flexible Pufferzone zwischen Kulturoberfläche und deren Halterung abgefangen werden. Dadurch können Materialien mit unterschiedlichen thermischen Ausdehnungskoeffizienten für das Substrat verwendet werden. Alternativ hierzu ist eine Verwendung desselben Materials für Kulturoberfläche und den Rest der Substrateinrichtung möglich. Dadurch sind die Volumenveränderungen dieselben und es kommt zu keinen Spannungen im Material.

Da eine Meniskus-Ausbildung das Medienvolumen über den Zellen im Randbereich des Substrats erhöht, kann der Winkel und das Material im Randbereich so angepasst werden, dass kein Meniskus mehr auftritt. Dadurch ist begünstigt, dass selbst im Randbereich eine optimale Vitrifikation der Zellen erfolgt.

Weitere Einzelheiten und Vorteile der Erfindung werden im Folgenden unter Bezug auf die beigefügten Zeichnungen beschrieben. Es zeigen:
- Figur 1:: eine schematische Schnittansicht einer ersten Ausführungsform der erfindungsgemäßen Substrateinrichtung im Kultivierungszustand;
- Figur 2:: eine schematische Schnittansicht einer Ausführungsform des erfindungsgemäßen Kryokonservierungsgeräts;
- Figur 3:: Schritte eines Kryokonservierungsverfahrens gemäß einer bevorzugten Ausführungsform der Erfindung;
- Figur 4:: vergrößerte Schnittansichten der Substratplattform der Substrateinrichtung gemäß Figur 1;
- Figur 5:: Schritte einer Rückgewinnung einer biologischen Probe nach einer Kryokonservierung;
- Figur 6:: eine schematische Schnittansicht einer weiteren Ausführungsform der erfindungsgemäßen Substrateinrichtung mit einer Zufuhreinrichtung;
- Figur 7:: schematische Schnittansichten einer weiteren Ausführungsform einer erfindungsgemäßen Substrateinrichtung mit einem Kammerrahmen;
- Figur 8:: schematische Schnittansichten einer weiteren Ausführungsform der erfindungsgemäßen Substrateinrichtung mit einer Schraubverbindung;
- Figur 9:: schematische Schnittansichten einer weiteren Ausführungsform einer erfindungsgemäßen Substrateinrichtung mit einer Mikrofluidikeinrichtung;
- Figur 10:: schematische Schnittansichten einer weiteren Ausführungsform der erfindungsgemäßen Substrateinrichtung mit einer Vielzahl von Teilkammern; und
- Figur 11:: eine schematische Illustration der herkömmlichen Kryokonservierung adhärenter biologischer Zellen (Stand der Technik).

Ausführungsformen der Erfindung werden im Folgenden unter Bezug auf die Merkmale der Substrateinrichtung und des Kryokonservierungsgeräts sowie die Schritte des Kryokonservierungsverfahren erläutert. Einzelheiten der Kultivierung biologischer Zellen im adhärenten Zustand oder in hängenden Tropfen, von Kultivierungsmedien, von der Verwendung von Kryoprotektiva, der Behandlungsprotokolle, der Überwachung der Konservierung und der Handhabung von Kühl- oder Heizmedien werden nicht beschrieben, soweit diese von herkömmlichen Techniken bekannt sind. Die Substrateinrichtung und das Kryokonservierungsgerät werden anhand schematischer Schnittansichten mit einer vertikalen Schnittebene (senkrecht zur Horizontalen) beispielhaft illustriert. Die geometrische Form der Substrateinrichtung bzw. des Kryokonservierungsgeräts, auch in von der Illustration abweichenden Raumrichtungen, kann in Abhängigkeit von der gewünschten Anwendung der Erfindung gewählt werden. Beispielsweise kann die Substrateinrichtung in Draufsicht (senkrecht zur Schnittebene in den Figuren) eine runde oder rechteckige Form haben. Des Weiteren kann die Zusammensetzung aus den ersten und zweiten Kammern die Gestalt eines Zylinders (Dosenform) haben. Bei der Kombination einer Vielzahl von ersten Kammern für die Probenkultivierung mit einer gemeinsamen Kammer für das Temperierungsmedium kann die Form und Anordnung der ersten Kammern ebenfalls in Abhängigkeit von den Bedingungen der gewünschten Anwendungen gewählt werden.

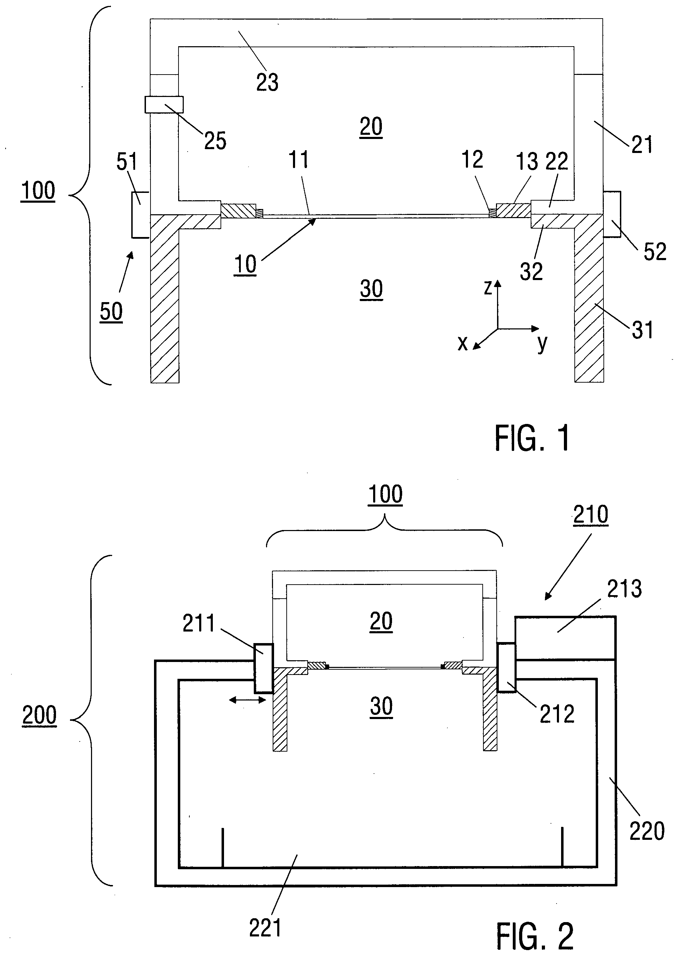

Die in Figur 1 gezeigte Ausführungsform der erfindungsgemäßen Substrateinrichtung 100 umfasst die Substratplattform 10, eine erste Kammer 20 und eine zweite Kammer 30. Die Substratplattform 10 bildet eine Trennwand zwischen den Innenräumen der ersten Kammer 20 und der zweiten Kammer 30.

Die Substratplattform 10 ist über eine Substrathalterung 12 und einen Ausgleichsabschnitt 13 mit einer Kammerwand 21 der ersten Kammer 20 verbunden. Die Kammerwand 21 der ersten Kammer 20 hat eine hohlzylinderförmige Gestalt, deren Zylinderachse die Symmetrieachse der Substrateinrichtung 100 bildet und in den Kultivierungs- und Temperierungszuständen (siehe unten) in vertikaler Richtung (z-Richtung) orientiert ist. Die zylinderförmige Kammerwand 21 der ersten Kammer 20 weist an ihrem zur zweiten Kammer 30 weisenden Ende eine Auskragung 22 auf, mit der der Ausgleichsabschnitt 13 verbunden ist. Am entgegengesetzten, von der zweiten Kammer 30 wegweisenden Ende weist die Kammerwand 21 der ersten Kammer 20 eine Aufnahme für einen Deckel 23 auf. Der Deckel 23 kann so mit der Kammerwand 21 der ersten Kammer 20 gekoppelt werden, dass zwischen dem Innenraum der ersten Kammer 20 und der Umgebung der Substrateinrichtung 100 ein Gasaustausch ausgeschlossen wird.

Die zweite Kammer 30 umfasst eine Kammerwand 31, deren Form und Größe an die Form und Größe der ersten Kammer 20 angepasst sind. Im dargestellten Beispiel ist die Kammerwand 31 der zweiten Kammer 30 ebenfalls hohlzylinderförmig mit einer an dem zur ersten Kammer 20 weisenden Rand radial nach innen vorspringenden Auskragung 32 geformt. Der Ausgleichsabschnitt 13 der Substratplattform 10 kann ausschließlich mit der Auskragung 22 der ersten Kammer 20, wie dargestellt mit beiden Auskragungen 22, 32 oder ausschließlich mit der Auskragung 32 der zweiten Kammer 30 verbunden sein. Die zweite Kammer 30 ist auf der von der Substratplattform 10 wegweisenden Seite offen. In einer abgewandelten Variante kann jedoch auch an der zweiten Kammer 30 ein Deckel vorgesehen sein.

Die Kammerwände 21, 31 der ersten und zweiten Kammern 20, 30 können aus verschiedenen Materialien, z. B. Kunststoff und/oder Metall, hergestellt und an den Auskragungen 22, 32 miteinander verbunden sein. Alternativ können die Kammerwände 21, 31 einstückig als integrale Komponente, z. B. aus Kunststoff oder Metall, hergestellt sein.

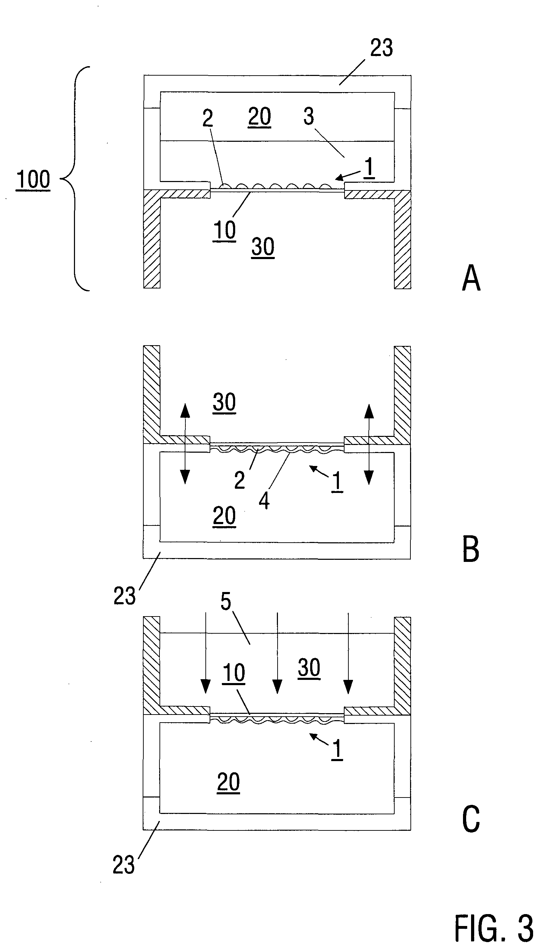

Die Substratplattform 10 umfasst eine ebene Platte aus Glas, Kunststoff oder Metall in Form einer Kreisscheibe. Die zu der ersten Kammer 20 weisende Oberseite der Glasplatte bildet die Kultivierungsfläche 11, die beispielsweise die freiliegende Glasoberfläche oder eine Glasoberfläche mit einer biokompatiblen Oberflächenschicht umfasst. Die Kultivierungsfläche 11 ist zur Aufnahme der Probe, die konserviert werden soll und mindestens eine biologische Zelle umfasst (siehe Figur 3), angepasst und kann insbesondere eine mit einer Zellkultur (z. B. hESCs, MEFs, iPS oder andere adhärent wachsende Zelltypen) versehene und/oder eine hydrophile Oberfläche umfassen.

Die Substrathalterung 12 ist ein kreisringförmiger Rahmen, in dem die Substratplattform 10 lösbar positioniert ist. Der Rahmen ist für eine flüssigkeitsdichte Ankopplung der Substratplattform 10 konfiguriert und umfasst z. B. eine Auflage für die Substratplattform und eine umlaufende Dichtlippe zur dichten Fixierung der aufgelegten Substratplattform 10. Der Austausch der Substratplattform 10, z. B. zum Zweck der Anpassung an eine bestimmte Kultivierungsaufgabe, kann durch Anheben der Dichtlippe und Entnahme der Substratplattform 10 erfolgen. Vorzugsweise ist die Substrathalterung 12 aus einem nachgiebigen Kunststoff, insbesondere Silikon-Gummi, oder aus Metall hergestellt.

Der Ausgleichsabschnitt 13 ist eine Dehnungsfuge zur Kompensation von Dimensionsänderungen der Teile der Substrateinrichtung 100 in Abhängigkeit von der Temperatur. Der Ausgleichsabschnitt 13 hat z. B. die Gestalt einer Ringscheibe, die aus Materialien hergestellt ist, die auf die angrenzenden Materialien einerseits der Substratplattform 11 und/oder der Substrathalterung 12 und andererseits der Auskragung 22, 32 und/oder der Kammerwand 21, 31 abgestimmt sind. Die Materialien sind so gewählt, dass sich deren thermische Ausdehnungen oder Schrumpfungen bei einer Temperaturänderung ergänzen bzw. aufheben. Schrumpft beispielsweise das Material der Kammerwand 21, 31 stärker als das der Substratplattform 11, so soll der Ausgleichsabschnitt 13 dies kompensieren (in diesem Beispiel also bei Kälte noch stärker schrumpfen als die Kammerwand 21, 31). Abweichend von der Illustration können die Substrathalterung 12 und der Ausgleichsabschnitt 13 einstückig als integrales Bauteil hergestellt sein. In diesem Fall dient dieses Bauteil sowohl als Substrathalterung als auch als Ausgleichsabschnitt.

Die Substrateinrichtung 100 hat in einer praktischen Ausführungsform beispielsweise die folgenden Maße: Durchmesser der Substratplattform: 20 mm, Dicke der Substratplattform: 180 µm (z. B. vom Typ "µ-dish" vom Hersteller ibidi GmbH, Deutschland), Außendurchmesser der Substrateinrichtung 100 in der x-y-Ebene: 35 mm, Dicke der ersten und zweiten Kammerwand 21, 31: 3 mm, Höhe der ersten und zweiten Kammerwand 21, 31 in z-Richtung: jeweils 10 mm, Höhe des Deckels 23 in z-Richtung: 4 mm. Die genannten Maße stellen lediglich Beispielangaben dar, die vom Fachmann in Abhängigkeit von den Anforderungen in der konkreten Anwendung gewählt werden können.

Auf der Außenseite der ersten und zweiten Kammern 20, 30, z. B. wie dargestellt in einer Ebene mit der Substratplattform 10, ist eine Kammerhalterung 50 angeordnet, die für eine Aufnahme der Substrateinrichtung 100 im Kryokonservierungsgerät, z. B. gemäß Figur 2, konfiguriert ist. Die Kammerhalterung 50 umfasst z. B. zwei auf der zylinderförmigen Außenseite der Substrateinrichtung 100 radial nach außen stehende Zapfen 51, 52, die entlang einer gemeinsamen Bezugslinie einander gegenüberliegend angeordnet sind.

Die Substrateinrichtung 100 kann gemäß einer Variante der Erfindung mit einem Druckausgleichsventil 25 ausgestattet sein, das in Figur 1 schematisch gezeigt ist. Das Druckausgleichsventil 25 ist in die Kammerwand 21 oder den Deckel 23 eingesetzt und dafür ausgelegt, einen eventuellen Überdruck in der ersten Kammer relativ zur Umgebung auszugleichen.

Figur 2 zeigt schematisch eine Ausführungsform des erfindungsgemäßen Kryokonservierungsgeräts 200, das zur Aufnahme einer Substrateinrichtung 100, z. B. gemäß Figur 1, und zur Implementierung des erfindungsgemäßen Kryokonservierungsverfahrens (siehe unten) angepasst ist. Das Kryokonservierungsgerät 200 umfasst eine Dreheinrichtung 210, mit der die Substrateinrichtung 100 gehaltert und gedreht werden kann, und einen Trägerrahmen 220, der für eine stabile Positionierung der Dreheinrichtung 210 mit der Substrateinrichtung 100 über einer Arbeitsfläche (nicht dargestellt), wie z. B. einer Tischplatte, konfiguriert ist. Der Trägerrahmen 220 ist ein z. B. aus Kunststoff und/oder Metall hergestelltes Bauteil, an dessen Unterseite eine Wanne 221 zur Aufnahme abfließender Kultivierungsflüssigkeit oder abfließenden Temperierungsmediums vorgesehen sein kann.

Die Dreheinrichtung 210 umfasst Drehlager 211, 212 die mit einem gegenseitigen Abstand an einander gegenüberliegenden Auslegern des Trägerrahmens 220 angeordnet sind. Die Drehlager 211, 212 nehmen die Zapfen 51, 52 der Kammerhalterung 50 (siehe Figur 1) auf. Hierzu kann z. B. eines der Drehlager 211 in axialer Richtung federnd verschiebbar angeordnet sein (siehe Doppelpfeil), so dass für das Einsetzen der Substrateinrichtung 100 in die Drehlager 211 deren gegenseitiger Abstand vergrößert werden kann. Die Dreheinrichtung 210 ist des Weiteren mit einer optional vorgesehenen, schematisch gezeigten Antriebseinrichtung 213 gezeigt. Die Antriebseinrichtung 213 umfasst z. B. einen Elektromotor, der für eine Drehung der Substrateinrichtung 100 ausgelegt und über Kraftübertragungselemente, wie z. B. Zahnräder, mit der Substrateinrichtung 100 verbunden ist.

Die Figur 3 illustriert schematisch Schritte des Verfahrens zur Kryokonservierung einer biologischen Probe 1 mit biologischen Zellen 2 gemäß einer Ausführungsform der Erfindung, bei der die biologischen Zellen 2 eine adhärente Zellkultur auf der Kultivierungsfläche der Substratplattform 10 bilden. Figur 3A zeigt die Bereitstellung der biologischen Probe 1 auf der Kultivierungsfläche der Substratplattform 10 in der ersten Kammer 20 der Substrateinrichtung 100 (siehe Figur 1), wobei die biologischen Zellen 2 von einer Kultivierungsflüssigkeit 3 umgeben sind. Bei dieser Ausführungsform der Erfindung erfolgt die Kultivierung der adhärenten Zellen in dem illustrierten Kultivierungszustand, in dem sich die erste Kammer 20 über der zweiten Kammer 30 befindet. Entsprechend kann die erste Kammer 20 mit der Kultivierungsflüssigkeit 3 gefüllt werden, während die zweite Kammer 30 nach unten offen und leer ist. Die Kultivierungsflüssigkeit 3 hat eine Zusammensetzung, die in Abhängigkeit von den Zelltypen und der konkreten Anwendung der Erfindung gewählt ist und Nährmedium und mindestens ein Kryoprotektivum, wie z. B. eine Zusammensetzung aus 20% DMSO, 20% Ethylenglykol und 300 mM Trehalose umfasst.

Die Kultivierung der biologischen Zellen 2 erfolgt gemäß einem vorbestimmten, zelltypspezifischen Kultivierungsprotokoll. Dabei kann der Deckel 23 der Substrateinrichtung 100 hermetisch geschlossen oder geöffnet sein. Die Verschließbarkeit der ersten Kammer 20 mit dem Deckel 23 ist insbesondere während des Transports der ersten Kammer 20 von Vorteil. Beispielsweise kann die Kultivierung an einem anderen Ort als die Kryokonservierung erfolgen, z. B. in einem Inkubationsgerät. Um dann die Kryokonservierung durchzuführen, kann die Substrateinrichtung 100 mit dem geschlossenen Deckel 23 z. B. zu einem Kryokonservierungsgerät gemäß Figur 2 transportiert werden.

In einem konkreten, experimentell getesteten Beispiel umfassen die Zellen 2 humane embryonale Stammzellkolonien (hESC Kolonien) die von einem Monolayer aus Mausfibroblasten umgeben sind, während die Kultivierungsflüssigkeit 3 aus 20% DMSO, 20% Ethylenglykol und 300 mM Trehalose in Standard-hESC Kulturmedium zusammengesetzt ist. Die Kultivierung gemäß Figur 3A kann sich in der Substrateinrichtung 100 über Stunden oder Tage erstrecken. Alternativ kann die Kultivierung der biologischen Zellen 2 auf der Substratplattform 10 zunächst in einem separaten Inkubationsgerät erfolgen. Nach Erreichen eines gewünschten Kultivierungsergebnisses kann dann die Substratplattform 10 in die Substrateinrichtung 100 eingesetzt werden.

In einem weiteren Teilschritt wird gemäß Figur 3B die Substrateinrichtung 100 um 180° um eine horizontale Drehachse gedreht. Die Substrateinrichtung 100 ist dann in einem Temperierungszustand, in dem sich die zweite Kammer 30 über der ersten Kammer 20 befindet. Die Probe 1 hängt im adhärenten Zustand an der nach unten weisenden Kultivierungsfläche der Substratplattform 10. Aufgrund der Oberflächenspannung der Kultivierungsflüssigkeit 3 bleibt auch im Temperierungszustand gemäß Figur 3B ein Flüssigkeitsfilm 4 erhalten, der die Zellen bedeckt. Vorteilhafterweise bleiben dadurch die Zellen 2 mit der Kultivierungsflüssigkeit in Gestalt des Flüssigkeitsfilms 4 in Kontakt, während das Volumen der Kultivierungsflüssigkeit durch die Filmbildung minimiert ist. Dadurch wird ein sprunghaftes Einfrieren der Probe 1 bei der nachfolgenden Zufuhr eines Kühlmediums 5 in die zweite Kammer 30 (siehe Figur 3C) unterstützt.

Die Kryokonservierung der Probe 1, insbesondere deren Vitrifikation, erfolgt durch das Einfüllen eines Kühlmediums 5, z. B. Flüssigstickstoff, in die zweite Kammer 30 (siehe Pfeile in Figur 3C). Durch die Substratplattform 10, welche gleichzeitig die Trennwand zum Innenraum der ersten Kammer 20 bildet, kommt es zum Wärmeaustausch, so dass die Probe 1 sprungartig auf - 196°C abgekühlt wird. Die Probe 1 wird vitrifiziert und bleibt bei Beibehaltung einer Temperatur unterhalb von - 130°C stabil. Die Stabilität der Probe ist insbesondere gewährleistet, wenn das Kühlmedium 5 in der zweiten Kammer erhalten bleibt. Dies ermöglicht insbesondere einen Transport der kryokonservierten Probe 1, der praktisch unbegrenzt möglich ist, solange das Kühlmedium 5 nachgefüllt wird. Bei einer Lagerung der Substrateinrichtung 100 im Dampf von flüssigem Stickstoff, z. B. in einer Gasphase in einem Stickstofftank, kann das Kühlmedium 5 in der zweiten Kammer 30 allmählich verdampfen, wobei jedoch die Probe 1 auch in der Gasphase bei rund - 170°C stabil vitrifiziert bleibt.

Die Figuren 3B und 3C zeigen den Temperierungszustand der Substrateinrichtung 100 mit der geschlossenen ersten Kammer 20. Entsprechend wird beim Drehen der Substrateinrichtung 100 vom Kultivierungszustand in den Temperierungszustand die verbleibende Kultivierungsflüssigkeit 3 durch den Deckel 23 in der ersten Kammer 20 zurückgehalten. Abweichend von der Darstellung kann der Deckel 23 im Temperierungszustand entfernt werden, so dass die Kultivierungsflüssigkeit 3, abgesehen von dem Flüssigkeitsfilm 4, vollständig abfließt.

Während in Figur 3 beispielhaft auf die Kultivierung und Kryokonservierung adhärenter Zellen Bezug genommen wird, können die Kultivierung und Kryokonservierung alternativ mit biologischen Zellen in hängenden Tropfen ("Hanging Droplet"-Kultivierung) durchgeführt werden. In diesem Fall wird bereits für die Kultivierung der Temperierungszustand der Substrateinrichtung eingestellt, wobei die zweite Kammer 30 über der ersten Kammer 20 angeordnet ist und die Kultivierungsfläche der Substratplattform nach unten, d. h. in Gravitationsrichtung weist. Tropfen der Kultivierungsflüssigkeit mit suspendierten Zellen hängen an der Kultivierungsfläche. Die Zufuhr und/oder der Austausch der Kultivierungsflüssigkeit, einschließlich der Kryoprotektiva, erfolgt mit Verfahren, wie sie von der herkömmlichen "Hanging Droplet"-Kultivierung bekannt sind. Zur Vitrifikation der Probe wird die zweite Kammer mit dem Kühlmedium beaufschlagt, so dass die Tropfen mit den Zellen sprungartig eingefroren werden.

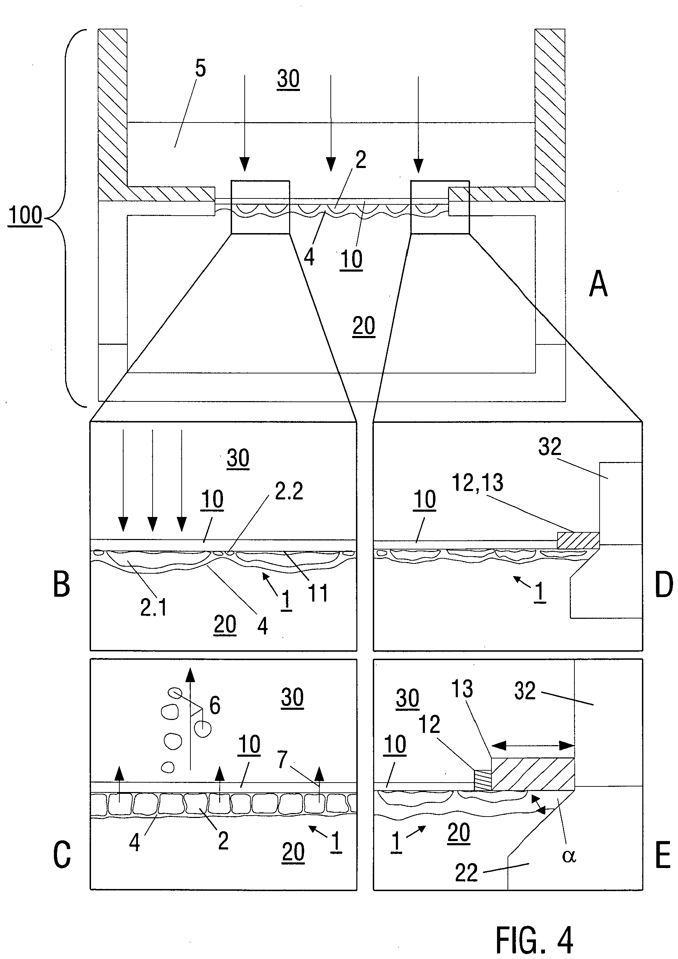

In Figur 4 sind weitere Einzelheiten der Substrateinrichtung 100 im Temperierungszustand gezeigt. Figur 4A zeigt die Substrateinrichtung 100 wie in Figur 3C mit der ersten Kammer 20, der zweiten Kammer 30 und der zwischen diesen eine Trennwand bildenden Substratplattform 10. Die biologischen Zellen 2 sind auf der nach unten weisenden Kultivierungsfläche der Substratplattform 10 adhärent angeordnet und mit dem Flüssigkeitsfilm 4 bedeckt. Die zweite Kammer 30 ist mit dem Kühlmedium 5 von oben (siehe Pfeile) befüllt, so dass die biologischen Zellen 2 sprunghaft abgekühlt und vitrifiziert wurden.

Figur 4B zeigt einen vergrößerten Ausschnitt der Substratplattform 10, auf deren nach unten, zu der ersten Kammer 20 hinweisenden Kultivierungsfläche 11 die Probe 1 mit biologischen Zellen, insbesondere Zellgruppen 2.1 und einzelne Zellen 2.2, verschiedener Zelltypen angeordnet ist. Beispielsweise umfassen die größeren Zellgruppen 2.1 (Zellkolonien) humane embryonale Stammzellkolonien, während die Zellen 2.2 embryonale Mausfibroblasten umfassen, die eine Zell-Monolage bilden. Die Zellgruppen 2.1 und Zellen 2.2 sind adhärent mit der Kultivierungsfläche 11 verbunden.

Bei Einfüllen des Kühlmediums 5 (siehe Pfeile in Figur 4B) in die zweite Kammer 30 wird die Substratplattform 10 sprunghaft abgekühlt. Da das Kühlmedium 5 von oben in die zweite Kammer 30 eingefüllt wird, kann das Leidenfrost'sche Phänomen minimiert werden. Gasblasen 6, die beim Auftreffen des Kühlmediums 5 auf der Substratplattform 10 entstehen (siehe abgewandelte Illustration in Figur 4C), steigen entgegengesetzt zur Gravitationsrichtung, d. h. nach oben auf, wobei das entsprechende Volumen durch nachströmendes Kühlmedium ersetzt wird. Die isolierende Wirkung von Gasblasen, wie sie bei herkömmlichen Kryokonservierungsverfahren auftritt, kann daher minimiert werden.

Figur 4C illustriert des Weiteren, dass durch die geringe Dicke der Substratplattform 10 der Abstand zwischen dem Kühlmedium 5 und den Zellen 2 minimiert wird. Die Wärme der Probe 1 mit Zellen 2 und dem Flüssigkeitsfilm 4 fließt sprungartig hin zum Kühlmedium 5 (siehe Pfeile 7 in Figur 4C).

Varianten der Befestigung der Substratplattform 10 an den Kammerwänden der ersten und zweiten Kammern 20, 30 sind in den Figuren 4D und 4E schematisch gezeigt. Die Substratplattform 10 ist z. B. mit der Substrathalterung 12 und dem Ausgleichsabschnitt 13 (als integrales Bauteil gezeigt) an der Auskragung 32 der Kammerwand der zweiten Kammer 30 fixiert (Figur 4D). Der Ausgleichsabschnitt 13 ermöglicht, dass insbesondere bei verschiedenen thermischen Ausdehnungskoeffizienten der Substratplattform 10 und der übrigen Wände der ersten und zweiten Kammern 20, 30 mechanische Spannungen an der Substratplattform 10 und der Substrathalterung 12 vermieden werden.

Figur 4E illustriert schematisch, dass die Auskragung 22 der Kammerwand der ersten Kammer 20 einen zum Inneren der Kammer 20 weisenden umlaufenden Vorsprung bildet, der die Substratplattform 10, die Substrathalterung 12 und/oder den Ausgleichsabschnitt 13 teilweise überragt. Der Winkel á zwischen der Ebene der Substratplattform 10 und dem Rand der Auskragung 22 entlang des Vorsprungs ist kleiner als 90°. Vorteilhafterweise wird damit erzielt, dass zwischen der Probe 1 und der Auskragung 22 kein Meniskus gebildet wird, sondern die Dicke der Probe 1 bis zur Auskragung 22 unverändert bleibt. Vorteilhafterweise sind damit sogar im Randbereich der Probe 1 gleichbleibende Kryokonservierungsbedingungen wie in der Mitte der Substratplattform 10 gegeben, so dass eine optimale Vitrifikation der Zellen 2 erfolgt.

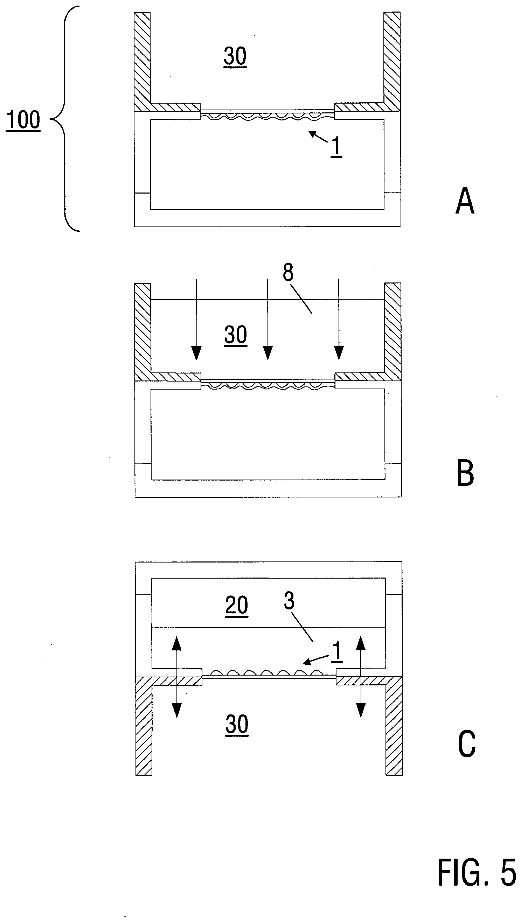

Die Figur 5 zeigt schematisch die Wiedergewinnung der Probe 1 nach der Kryokonservierung. Da das flüssige Kühlmedium, wie z. B. Flüssigstickstoff, bei einer Lagerung in der Gasphase eines Stickstofftanks verdampft, ist die zweite Kammer 30 während der Kryokonservierung typischerweise leer (Figur 5A). In dieser Situation kann die Substrateinrichtung 100 aus dem Stickstofftank entnommen werden, um die Probe 1 aufzutauen. Hierzu wird ein Heizmedium 8, wie z. B. Wasser mit einer Temperatur von 37°C, in die zweite Kammer 30 von oben (siehe Pfeile in Figur 5B) eingefüllt. Das Auftauen der Probe kann wie die Kryokonservierung mit hoher Geschwindigkeit erfolgen, so dass eine Eiskristallbildung in der Probe 1 auch während des Auftauens vermieden wird.

Anschließend wird gemäß Figur 5C die Substrateinrichtung 100 wieder in den Kultivierungszustand gedreht (siehe Doppelpfeile), in dem die erste Kammer 20 über der zweiten Kammer 30 angeordnet ist. Die zweite Kammer 30 wird geleert, während in die erste Kammer 20 erneut eine Kultivierungsflüssigkeit 3 eingefüllt wird. Mit der Kultivierungsflüssigkeit 3 erfolgt beispielsweise eine Waschung der Probe 1 und eine weitere Kultivierung. Weitere Verfahrensschritte, wie z. B. ein Passagieren oder eine weitere Vitrifikation können sich anschließen.

Alternativ zu den in Figur 5 gezeigten Schritten kann beim Auftauen vorgesehen sein, dass das Heizmedium im Kultivierungszustand der Substrateinrichtung 100 in die erste Kammer 20 gefüllt wird. In diesem Fall kann das Heizmedium eine auf 37°C erwärmte Kultivierungsflüssigkeit sein.

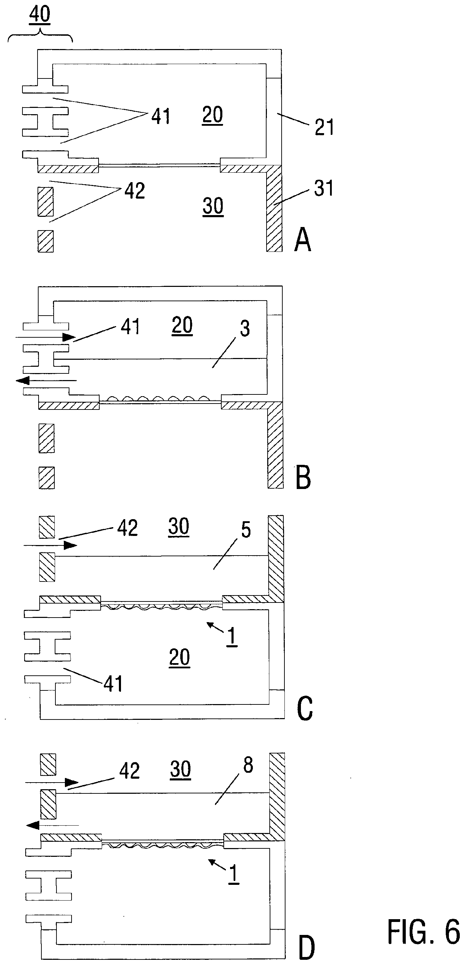

Gemäß einer weiteren Ausführungsform der Erfindung kann die Substrateinrichtung 100 mit einer Zufuhreinrichtung 40 ausgestattet sein, wie beispielhaft in Figur 6 gezeigt ist. Beim dargestellten Beispiel umfasst die Zufuhreinrichtung 40 eine Vielzahl von Medienleitungen 41, 42 die jeweils mit der ersten Kammer 20 oder der zweiten Kammer 30 verbunden sind. Die Medienleitungen 41, 42 sind z. B. Schläuche oder Rohre (nicht vollständig gezeigt), die mit Flüssigkeitsreservoiren und Pumpen verbunden sind, und sie ermöglichen insbesondere eine automatisierte Zufuhr von Kultivierungsflüssigkeiten in die erste Kammer 20 und eine automatisierte Zufuhr von Kühl- oder Heizmedien in die zweite Kammer 30. Die Medienleitungen 41, 42 (oder zumindest die illustrierten Enden oder Anschlüsse der Medienleitungen) sind in die Kammerwände 21, 31 der ersten bzw. zweiten Kammern 20, 30 integriert. Alternativ können Medienleitungen auch mit einem Deckel der ersten oder zweiten Kammern verbunden sein.

Gemäß Figur 6B erfolgt im Kultivierungszustand durch die Medienleitungen 41 der ersten Kammer 20 die Zufuhr mindestens einer Kultivierungsflüssigkeit 3. Beispielsweise kann gemäß einem vorbestimmten Kultivierungsprotokoll eine Folge von Nährmedien und/oder Kryoprotektiva mit vorbestimmter Zusammensetzung und/oder Konzentration in die erste Kammer 20 eingeführt werden.

Zum Einfrieren der Probe 1 wird die Substrateinrichtung in den Temperierungszustand gedreht, in dem sich die zweite Kammer 30 oberhalb von der ersten Kammer 20 befindet (Figur 6C). In dieser Situation wird über die Medienleitungen 42 der zweiten Kammer 30 Kühlmittel in den Innenraum der zweiten Kammer 30 eingefüllt. Der Füllstand des Kühlmittels 5 in der zweiten Kammer 30 kann durch eine geeignete Steuerung der Zu- und Abfuhr von Kühlmittel eingestellt werden. Gleichzeitig wird die erste Kammer 20 über die Medienleitungen 41 der ersten Kammer 20 geleert. Zum Auftauen der Probe 1 ist gemäß Figur 6D vorgesehen, dass die zweite Kammer 30 über die Medienleitungen 42 mit einem Heizmedium 8, wie z. B. Wasser, gefüllt wird.

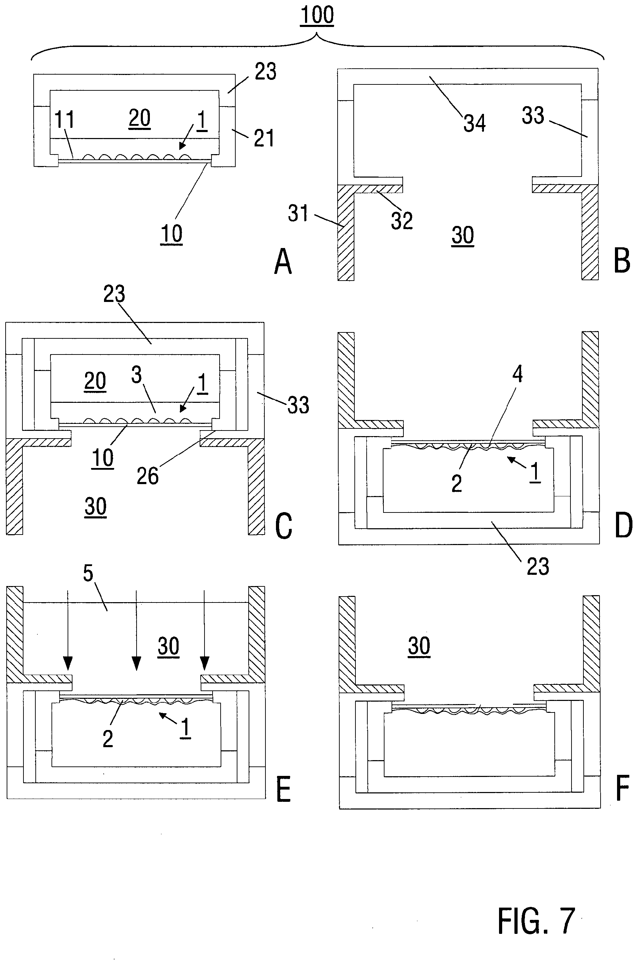

Abweichend von der Ausführungsform gemäß Figur 1 können die ersten und zweiten Kammern 20, 30 der erfindungsgemäßen Substrateinrichtung 100 lösbar miteinander verbunden sein, wie beispielhaft in Figur 7 gezeigt ist. Die erste Kammer 20 (Figur 7A) ist ein Behälter, dessen Innenraum durch die Substratplattform 10, die Kammerwand 21 und den Deckel 23 umschlossen ist. Die Kultivierungsfläche 11 der Substratplattform 10 ist zur Aufnahme der Probe 1 im adhärenten Zustand oder in hängenden Tropfen zum Innenraum der ersten Kammer 20 weisend angeordnet, während die zur Kultivierungsfläche 11 entgegengesetzte Oberfläche der Substratplattform 10 nach außen frei liegt. Die zweite Kammer 30 (Figur 7B) umfasst eine Zusammensetzung aus einer Kammerwand 31 mit radial nach innen vorspringender Auskragung 32 und einem Kammerrahmen 33, der zur lösbaren Aufnahme der ersten Kammer 20 eingerichtet ist. Der Kammerrahmen 33 ist eine zylinderförmige Aufnahme, deren Innendurchmesser gleich dem Außendurchmesser der ersten Kammer 20 ist. Auf der von der zweiten Kammer 30 wegweisenden Seite ist ein Rahmendeckel 34 vorgesehen, mit dem die erste Kammer 20 im Kammerrahmen 33 fixierbar ist. Die zur zweiten Kammer 30 weisende Seite des Kammerrahmens 33 weist eine Öffnung auf, durch welche die Substratplattform 10 hin zum Innenraum der zweiten Kammer 30 frei liegt.

Der in den Figuren 7A und 7B gezeigte zweiteilige Aufbau der Substrateinrichtung 100 hat den Vorteil, dass die zweite Kammer 30 (die Vitrifikations-Kammer) als Ummantelung für die erste Kammer 20 (die Kultivierungskammer) mehrfach verwendbar ist. Die erste Kammer 20 kann in ihrer Gestalt an die optimale Kultivierung, z. B. in einem Inkubationsgerät, angepasst sein. Die zweite Kammer 30 wird mit der ersten Kammer 20 erst dann gekoppelt, wenn die gewünschte Kryokonservierung durchgeführt werden soll. Des Weiteren können kommerziell verfügbare Kultivierungssubstrate für die erste Kammer verwendet werden, falls die zweite Kammer 30 mit dem Kammerrahmen 33 geeignet an die Geometrie des jeweiligen Kultursubstrats angepasst ist.

Die Ausführungsform der Substrateinrichtung 100 gemäß den Figuren 7A und 7B hat den Vorteil, dass die Kultivierung und Beobachtung, z. B. Mikroskopuntersuchung, der Probe 1 ohne die zweite Kammer 30 und ohne den Kammerrahmen 33 erfolgen können. Dies bietet zusätzliche Freiheitsgrade bei der Vorbereitung der Probe 1 für die Kryokonservierung und/oder die Auswahl geeigneter Proben mit biologischen Zellen in einem vorbestimmten Kultivierungsstadium.

Die Figuren 7C bis 7F zeigen die Schritte der erfindungsgemäßen Kryokonservierung unter Verwendung der Substrateinrichtung 100 gemäß den Figuren 7A und 7B. Gemäß Figur 7C ist die erste Kammer 20 mit der Substratplattform 10, der Probe 1 und der Kultivierungsflüssigkeit 3 in den Kammerrahmen 33 eingesetzt und fixiert. Bei Bedarf kann an der Kontaktfläche 26 schen der ersten Kammer 20 und dem Kammerrahmen 33 eine Dichtung (nicht dargestellt) vorgesehen sein, um ein Eindringen des Kühlmediums von der zweiten Kammer 30 in die Umgebung der ersten Kammer 20, insbesondere zu deren Deckel 23, zu verhindern.

Vor Beginn der Vitrifikation wird die Substrateinrichtung um 180° relativ zu der Ebene der Substratplattform 10 gedreht, so dass die Probe 1 mit den Zellen 2 und dem Flüssigkeitsfilm 4 nach unten hängend angeordnet sind. Die überschüssige Kultivierungsflüssigkeit wird in dem nach unten weisenden Deckel 23 der ersten Kammer aufgefangen oder über eine Medienleitung (nicht dargestellt) abgeleitet (Figur 7D).

Anschließend erfolgt die Zufuhr des Kryomediums 5, z. B. von Flüssigstickstoff, in die zweite Kammer 30 (Figur 7E). Im Ergebnis wird die Wärme von der Substratplattform 10 und der Probe 1 sprungartig abtransportiert, und die Probe 1 mit den biologischen Zellen 2 wird vitrifiziert. Die weitere Lagerung kann in der Gasphase über flüssigem Stickstoff im Stickstofftank erfolgen, wobei das flüssige Kühlmedium aus der zweiten Kammer 30 verdampfen kann (Figur 7F).

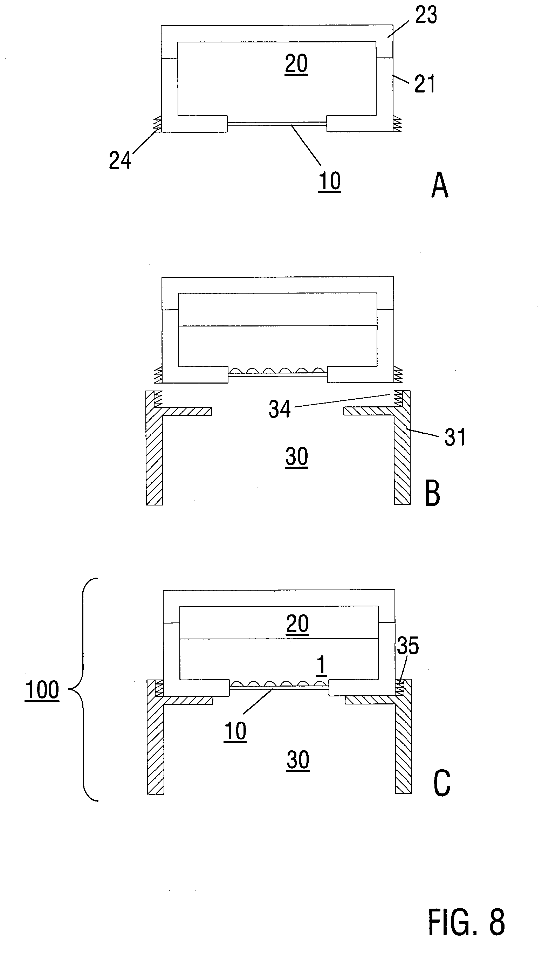

Eine weitere Variante der mehrteiligen Substrateinrichtung 100 ist in Figur 8 illustriert. Die erste Kammer 20 mit der Substratplattform 10, der Kammerwand 21 und dem Deckel 23 ist als verschließbarer Behälter aufgebaut. Auf der Außenseite der ersten Kammer 20, insbesondere auf der Außenseite der Kammerwand 21 ist ein Außengewinde 24 vorgesehen, das mit einem Innengewinde 34 an der Kammerwand 31 der zweiten Kammer 30 zur Bildung einer Schraubverbindung 35 zusammenwirkt (Figur 8B). Zur Aufnahme im Kryokonservierungsgerät 200 (Figur 2) kann die zweite Kammer 30 auf ihrer Außenseite eine Kammerhalterung aufweisen, wie sie oben unter Bezug auf Figur 1 beschrieben wurde. Im zusammengesetzten Zustand der ersten Kammer 20 und der zweiten Kammer 30 (Figur 8C) liegt die Substratplattform 10 zum Innenraum der zweiten Kammer 30 frei, während die Innenseite der Substratplattform 10, wie oben beschrieben, die Kultivierungsfläche für die Probe 1 im Innenraum der ersten Kammer 20 bildet. Im zusammengesetzten Zustand kann die Substrateinrichtung 100 für die Vitrifikation der Probe 1 verwendet werden, wie dies oben beschrieben ist.

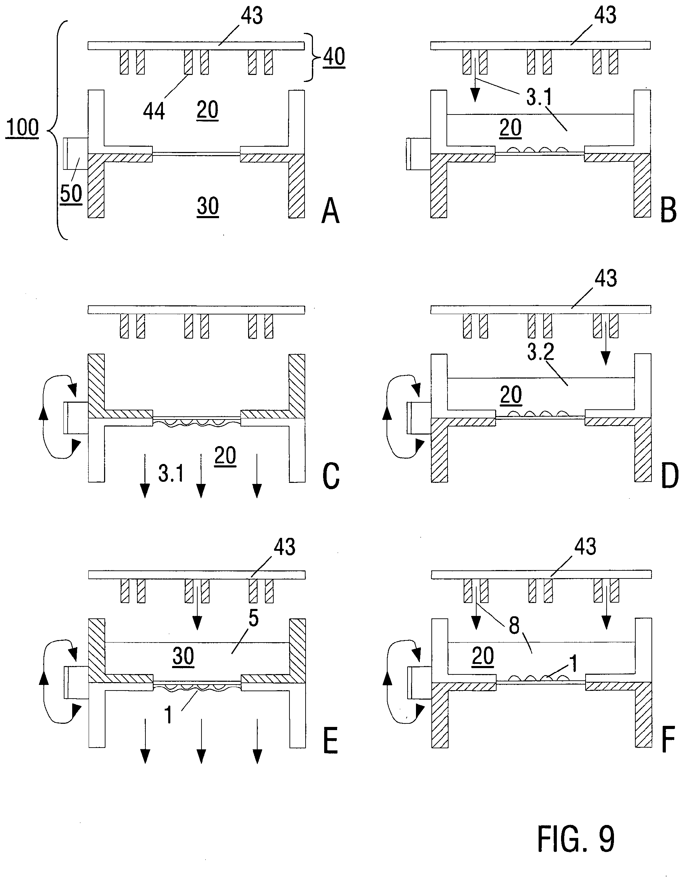

Figur 9 zeigt eine weitere Ausführungsform der erfindungsgemäßen Substrateinrichtung 100, bei der die Zufuhreinrichtung 40 zur Zuführung der mindestens einen Kultivierungsflüssigkeit eine Mikrofluidikeinrichtung 43 (oder: Medienzugabechip) umfasst. Die Mikrofluidikeinrichtung 43 enthält mikrofluidische Elemente 44, wie z. B. Medienleitungen, Ventile, Flüssigkeitsreservoire und/oder Pumpen. Mit der Mikrofluidikeinrichtung 43 können gezielt, insbesondere automatisiert, Kultivierungsmedien, Kryoprotektiva, oder Wasser in den Innenraum der ersten Kammer 20 und/oder der zweiten Kammer 30 zugeführt werden.

Gemäß der Erfindung ist es nicht zwingend erforderlich, dass die Mikrofluidikeinrichtung 43 mit der Substrateinrichtung 100 fest verbunden ist. Vielmehr kann die Mikrofluidikeinrichtung 43 von den ersten und zweiten Kammern 20, 30 getrennt angeordnet sein, wie in Figur 9 gezeigt ist. In diesem Fall erfüllt die Mikrofluidikeinrichtung 43 vorzugsweise eine Doppelfunktion, insbesondere zur Zufuhr der Kultivierungsflüssigkeit und zur Zufuhr des Kühlmediums. Die Mikrofluidikeinrichtung 43 kann z. B. oberhalb der Substrateinrichtung 100 ortsfest, z. B. als Teil des Kultivierungsgeräts 200 gemäß Figur 2, vorgesehen sein, um Flüssigkeiten jeweils in die erste oder zweite Kammer 20, 30, die sich im Kultivierungs- oder Temperierungszustand an der Oberseite der Substrateinrichtung 100 befindet, zuzuführen. Die in Figur 9 gezeigte Ausführungsform der Erfindung hat den besonderen Vorteil, dass die Schritte der Zuführung mindestens einer Kultivierungsflüssigkeit 3.1, 3.2, des Kühlmediums und des Heizmediums vollständig automatisierbar sind.

Alternativ kann mindestens eine Mikrofluidikeinrichtung in einen Deckel und/oder eine Kammerwand der ersten und/oder zweiten Kammer integriert sein. Beispielsweise kann eine weitere Mikrofluidikeinrichtung zur Zufuhr des Kühlmediums, z. B. Flüssigstickstoff, in die zweite Kammer vorgesehen sein.

Die Figuren 9B bis 9F illustrieren schematisch den Ablauf der Kryokonservierung mit der Substrateinrichtung 100 und der Mikrofluidikeinrichtung 43. Gemäß Figur 9B wird, wenn sich die Substrateinrichtung im Kultivierungszustand befindet, mit der Mikrofluidikeinrichtung 43 eine erste Kultivierungsflüssigkeit 3.1 in die erste Kammer 20 geleitet. Anschließend wird die Substrateinrichtung an der Kammerhalterung 50 um 180° gedreht, so dass die erste Kammer 20 nach unten weist und die erste Kultivierungsflüssigkeit 3.1 aus der ersten Kammer 20 abläuft (Figur 9C). Nach einer erneuten Drehung um 180°, nach der die erste Kammer 20 wieder auf der Oberseite der Substrateinrichtung angeordnet ist, wird mit der Mikrofluidikeinrichtung 43 eine weitere Kultivierungsflüssigkeit 3.2 in die erste Kammer 20 eingefüllt (Figur 9D). Schließlich folgt gemäß Figur 9E die Vitrifikation der Probe 1. Hierzu wird die Substrateinrichtung 100 nochmals um 180° gedreht, so dass die zweite Kammer 30 auf der Oberseite der Substrateinrichtung angeordnet ist. Mit der Mikrofluidikeinrichtung 43 wird Kühlmedium 5, wie z. B. Flüssigstickstoff, in die zweite Kammer 30 eingefüllt.

Schließlich illustriert Figur 9F schematisch die Rückgewinnung der Probe 1, wobei mit der Mikrofluidikeinrichtung 43 ein Heizmedium 8, wie z. B. erwärmtes Wasser, und Waschsubstanzen zum Auswaschen von Kryoprotektiva in die erste Kammer 20 zugegeben werden.

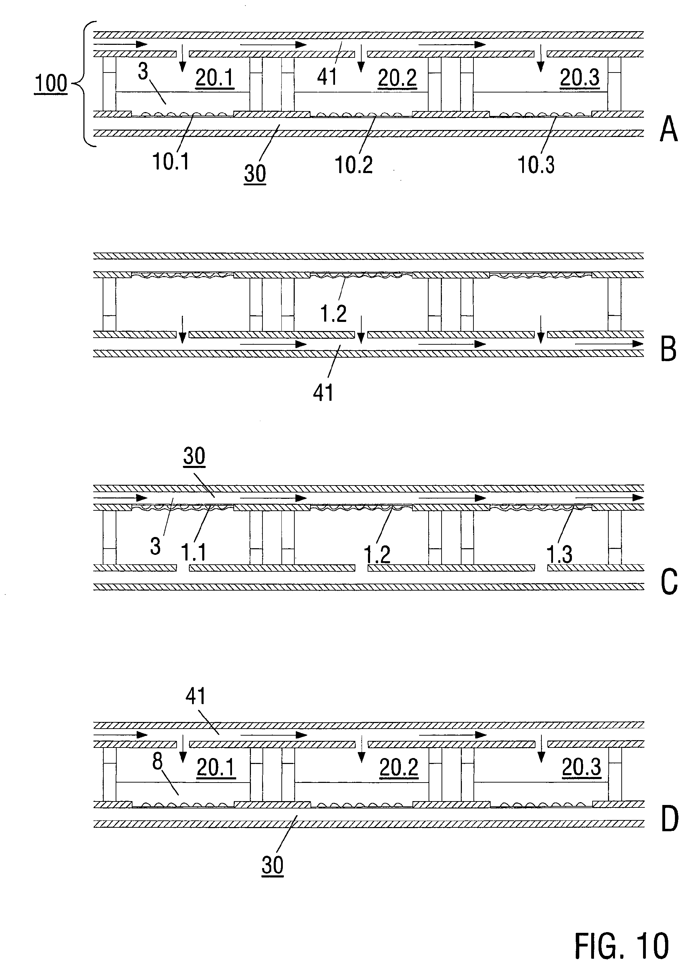

Figur 10 illustriert eine weitere Ausführungsform der erfindungsgemäßen Substrateinrichtung 100, bei der die erste Kammer eine Vielzahl von Teilkammern 20.1, 20.2, 20.3,... umfasst, die jeweils eine Substratplattform 10.1, 10.2, 10.3, ... aufweisen und an eine zweite Kammer 30 angrenzend angeordnet sind. Eine erste Medienleitung 41 ist vorgesehen, um eine Kultivierungsflüssigkeit 3 in die Teilkammern 20.1, 20.2, 20.3, ... zu leiten (Figur 10A) oder aus diesen Teilkammern abzuführen (Figur 10B). Zur Zuführung und Ableitung mindestens einer Kultivierungsflüssigkeit kann die Substrateinrichtung 100 jeweils um 180° gedreht werden, wie dies unter Bezug auf die oben gezeigten Ausführungsformen beschrieben wurde. Zur Vitrifikation der Proben 1.1, 1.2, 1.3,... wird ein Kühlmedium 5, z. B. Flüssigstickstoff, über eine zweite Medienleitung in die zweite Kammer 30 geleitet (Figur 10C), wenn sich die Substrateinrichtung 100 in dem Temperierungszustand befindet. Figur 10E illustriert entsprechend die Rückgewinnung der Proben, für die durch die erste Medienleitung 41 im Kultivierungsstand der Substrateinrichtung 100 ein Heizmedium 8 in die Teilkammern 20.1, 20.2, 20.3, ... geleitet wird.

Die Ausführungsform der Substrateinrichtung 100 gemäß Figur 10 bildet vorteilhafterweise einen kompakten Aufbau, bei dem sämtliche Zu- und Ableitungen in dem Gerät enthalten und auf die Eigenschaften der Substratplattformen abgestimmt sind. Vorteilhafterweise können Medienwechsel, Medienzugaben, die Inkubation, Vitrifikation und das Auftauen vollautomatisiert und mit mehreren Proben gleichzeitig durchgeführt werden. Dadurch entfallen ein Öffnen und ein manuelles Entnehmen oder Zugeben von Medien oder Kryoprotektiva.

Die in der vorstehenden Beschreibung, den Zeichnungen und den Ansprüchen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Substrateinrichtung (100), die zur Kryokonservierung einer biologischen Probe (1) mit biologischen Zellen (2, 2.1, 2.2) eingerichtet ist, umfassend
- eine Substratplattform (10) mit einer Vorderseite und einer Rückseite, wobei die Vorderseite der Substratplattform (10) eine Kultivierungsfläche (11) zur Aufnahme der biologischen Probe (1) bildet,
- eine erste Kammer (20), in der die Kultivierungsfläche (11) der Substratplattform (10) enthalten ist, wobei die erste Kammer (20) zur Aufnahme einer Kultivierungsflüssigkeit (3) eingerichtet ist, und
- eine zweite Kammer (30), die zur Aufnahme eines Temperierungsmediums (5) eingerichtet ist, wobei
- die erste Kammer (20) und die zweite Kammer (30) aneinander grenzend miteinander verbunden sind, und
- die Substratplattform (10) eine Trennwand zwischen der ersten Kammer (20) und der zweiten Kammer (30) bildet, wobei die Rückseite der Substratplattform (10) zu der zweiten Kammer (30) weist,
**dadurch gekennzeichnet dass**
- die Substrateinrichtung (100) eine Kammerhalterung (50) umfasst, die für eine schwenkfähige Aufnahme der Substrateinrichtung (100) in einem Kryokonservierungsgerät eingerichtet ist, wobei
- die Kammerhalterung (50) zwei in einer Ebene parallel zur Ausdehnung der Substratplattform (10) angeordnete Trägerelemente umfasst, die mit dem Kryokonservierungsgerät koppelbar sind, so dass die Substrateinrichtung (100) im Kryokonservierungsgerät um eine Querachse drehbar ist.

2. Substrateinrichtung gemäß Anspruch 1, bei der
- die Rückseite der Substratplattform (10) in der zweiten Kammer (30) freiliegt, und
- die Substratplattform (10) mit einer Dicke und einer Wärmeleitfähigkeit gebildet ist, die bei Benetzung der Rückseite der Substratplattform (10) mit einem ersten Temperierungsmedium (5) mit einer Temperatur unter - 120 °C, insbesondere mit flüssigem Stickstoff, eine Vitrifikation der biologischen Probe (1) an der Kultivierungsfläche (11) erlaubt.

3. Substrateinrichtung gemäß einem der vorhergehenden Ansprüche, bei der die Substratplattform (10) mindestens eine der Eigenschaften aufweist, die umfassen
- die Substratplattform (10) hat eine Dicke, die geringer ist als 200 µm, insbesondere geringer als 100 µm,
- die Substratplattform (10) ist aus Glas, Kunststoff, Halbleitermaterial oder Metall gebildet, und
- die Substratplattform (10) ist aus einem transparenten Material gebildet.

4. Substrateinrichtung gemäß einem der vorhergehenden Ansprüche, bei der
- die Substratplattform (10) über eine flüssigkeitsdichte Substrathalterung (12) lösbar mit der ersten oder der zweiten Kammer (20, 30) verbunden ist.

5. Substrateinrichtung gemäß einem der vorhergehenden Ansprüche, die mindestens eines der Merkmale aufweist, die umfassen
- die Substratplattform (10) ist über einen Ausgleichsabschnitt (13) mit der ersten oder der zweiten Kammer (20, 30) verbunden, wobei der Ausgleichsabschnitt (13) zur Aufnahme temperaturabhängiger Spannungen zwischen der Substratplattform (10) und der ersten oder der zweiten Kammer (20, 30) eingerichtet ist, und
- die erste Kammer (20) weist ein Druckausgleichsventil (25) auf, das zum Druckausgleich zwischen der ersten Kammer (20) und deren Umgebung eingerichtet ist.

6. Substrateinrichtung gemäß einem der Ansprüche 1 bis 4, bei der
- die Substratplattform (10) ein integraler Bestandteil der ersten oder der zweiten Kammer (20, 30) ist.

7. Substrateinrichtung gemäß einem der vorhergehenden Ansprüche, die umfasst
- eine Zufuhreinrichtung (40), die zur Zuführung der Kultivierungsflüssigkeit (3) und/oder des Temperierungsmediums (5) eingerichtet ist.

8. Substrateinrichtung gemäß Anspruch 7, bei der
- die Zufuhreinrichtung (40) mindestens eines von mindestens einer Medienleitung (41, 42) und einer Mikrofluidikeinrichtung (43) umfasst.

9. Substrateinrichtung gemäß einem der vorhergehenden Ansprüche, bei der
- die erste und die zweite Kammer (20, 30) lösbar miteinander verbunden sind.

10. Substrateinrichtung gemäß Anspruch 9, bei der
- die zweite Kammer (30) mit einem Kammerrahmen (33) fest verbunden ist, der zur lösbaren Aufnahme der ersten Kammer (20) eingerichtet ist.

11. Substrateinrichtung gemäß Anspruch 9 oder 10, bei der
- die erste Kammer (20) über eine Schraubverbindung (35) mit der zweiten Kammer (30) verbunden ist.

12. Substrateinrichtung gemäß einem der vorhergehenden Ansprüche, bei der
- die erste Kammer (20) mehrere Teilkammern (20.1, 20.2, 20.3, ...) umfasst, die an die zweite Kammer (30) grenzend angeordnet sind, wobei die Substratplattform (10) eine Trennwand zwischen den Teilkammern (20.1, 20.2, 20.3, ...) und der zweiten Kammer (30) bildet.

13. Kryokonservierungsgerät (200), umfassend
- mindestens eine Substrateinrichtung (100) gemäß einem der vorhergehenden Ansprüche, und
- eine Dreheinrichtung (210), die zur Aufnahme der mindestens einen Substrateinrichtung (100) konfiguriert ist, wobei
- die Substrateinrichtung (100) mit der Dreheinrichtung (210) zwischen einem Kultivierungszustand, in dem die Substratplattform (10) einen Boden der ersten Kammer (20) bildet, und einem Temperierungszustand verschwenkbar ist, in dem die Substratplattform (10) einen Boden der zweiten Kammer (30) bildet.

14. Verfahren zur Kryokonservierung einer biologischen Probe (1) mit biologischen Zellen (2, 2.1, 2.2) mit einer Substrateinrichtung (100) oder einem Kryokonservierungsgerät (200) gemäß einem der vorhergehenden Ansprüche, umfassend die Schritte
- Bereitstellung der biologischen Probe (1) an der Kultivierungsfläche (11) der Substratplattform (10) in der ersten Kammer (20), wobei die biologischen Zellen (2, 2.1, 2.2) von einer Kultivierungsflüssigkeit (3) umgeben sind, und
- Aufnahme eines Kühlmediums (5) in die zweite Kammer (30), die an die erste Kammer (20) angrenzt und mit dieser so verbunden ist, dass die Substratplattform (10) die Trennwand zwischen der ersten Kammer (20) und der zweiten Kammer (30) bildet, wobei die Temperatur der Substratplattform (10) abgesenkt und die biologische Probe (1) in einen gefrorenen Zustand überführt wird, wobei
die Kultivierung der biologischen Zellen (2, 2.1, 2.2) die Schritte umfasst
- Bereitstellung der ersten Kammer (20) und der zweiten Kammer (30) in einem Kultivierungszustand, in dem die Substratplattform (10) einen Boden der ersten Kammer (20) bildet und die Kultivierungsflüssigkeit (3) in die erste Kammer (20) gefüllt ist, und
- Aufnahme der biologischen Zellen (2, 2.1, 2.2) im adhärenten Zustand auf der Kultivierungsfläche (11) der Substratplattform (10), wobei
- vor der Aufnahme des Kühlmediums (5) in der zweiten Kammer ein Verschwenken der ersten Kammer (20) und der zweiten Kammer (30) in einen Temperierungszustand, in dem die Substratplattform (10) einen Boden der zweiten Kammer (30) bildet, und ein Abfließen der Kultivierungsflüssigkeit (3) aus der ersten Kammer erfolgen.

15. Verfahren gemäß Anspruch 14, bei dem die Kultivierung der biologischen Zellen (2, 2.1, 2.2) die Schritte umfasst
- Bereitstellung der ersten Kammer (20) und der zweiten Kammer (30) in einem Temperierungszustand, in dem die Substratplattform (10) einen Boden der zweiten Kammer (30) bildet, und
- Aufnahme der biologischen Zellen (2) in Tropfen der Kultivierungsflüssigkeit (3), die an der Kultivierungsfläche (11) der Substratplattform (10) hängen.

16. Verfahren gemäß einem der Ansprüche 14 bis 15, bei dem
- die Temperatur der Substratplattform (10) mit einer derartigen Kühlrate abgesenkt wird, dass die biologische Probe (1) vitrifiziert wird.

17. Verfahren gemäß einem der Ansprüche 14 bis 16, mit dem Schritt
- Aufnahme eines Heizmediums (8) in die zweite Kammer (30), wobei die Temperatur der Substratplattform (10) erhöht und die biologische Probe (1) in einen aufgetauten Zustand überführt wird.

## Claims

1. Substrate device (100), adapted for the cryopreservation of a biological sample (1) including biological cells (2, 2.1, 2.2), comprising
- a substrate platform (10) having a front side and a back side, the front side of the substrate platform (10) forming a cultivation surface (11) for receiving the biological sample (1),
- a first chamber (20), in which the cultivation surface (11) of the substrate platform (10) is included, the first chamber (20) being adapted for receiving a cultivation liquid (3), and
- a second chamber (30), which is adapted for receiving a temperature control medium (5), wherein
- the first chamber (20) and the second chamber (30) are connected to each other in an adjacent manner, and
- the substrate platform (10) forms a separating wall between the first chamber (20) and the second chamber (30), the back side of the substrate platform (10) facing towards the second chamber (30),
**characterized in that**
- the substrate device (100) comprises a chamber holder (50), which is adapted for receiving the substrate device (100) in a cryopreservation apparatus in a pivotable manner, wherein
- the chamber holder (50) comprises two supporting elements arranged in a plane parallel to the extension of the substrate platform (10), which supporting elements can be coupled to the cryopreservation apparatus so that the substrate device (100) in the cryopreservation apparatus can be rotated about a lateral axis.

2. Substrate device according to claim 1, wherein
- the back side of the substrate platform (10) is exposed in the second chamber (30), and
- the substrate platform (10) is formed with a thickness and a thermal conductivity which, when the back side of the substrate platform (10) is wetted with a first temperature control medium (5) at a temperature below -120°C, in particular with liquid nitrogen, allow vitrification of the biological sample (1) on the cultivation surface (11).

3. Substrate device according to one of the preceding claims, wherein the substrate platform (10) has at least one of the following features:
- the substrate platform (10) has a thickness below 200 µm, in particular below 100 µm,
- the substrate platform (10) is made of glass, plastic, semiconductor material or metal, and
- the substrate platform (10) is made of a transparent material.

4. Substrate device according to one of the preceding claims, wherein
- the substrate platform (10) is detachably connected to the first or second chamber (20, 30) via a liquid-tight substrate holder (12).

5. Substrate device according to one of the preceding claims, which has at least one of the following features:
- the substrate platform (10) is connected to the first or second chamber (20, 30) via a compensating section (13), the compensating section (13) being configured for absorbing temperature-dependent stresses between the substrate platform (10) and the first or second chamber (20, 30), and
- the first chamber (20) has a pressure equalizing valve (25) which is adapted for equalizing the pressure between the first chamber (20) and its surroundings.

6. Substrate device according to one of claims 1 to 4, wherein
- the substrate platform (10) is an integral component of the first or second chamber (20, 30).

7. Substrate device according to one of the preceding claims, which comprises
- a delivery device (40), which is adapted for delivering the cultivation liquid (3) and/or the temperature control medium (5).

8. Substrate device according to claim 7, wherein
- the delivery device (40) comprises at least one of at least one media line (41, 42) and a micro-fluidic unit (43).

9. Substrate device according to one of the preceding claims, wherein
- the first and second chambers (20, 30) are detachably connected to each other.

10. Substrate device according to claim 9, wherein
- the second chamber (30) is firmly connected to a chamber frame (33), which is configured for detachably receiving the first chamber (20).

11. Substrate device according to claim 9 or 10, wherein
- the first chamber (20) is connected to the second chamber (30) via a screw joint (35).

12. Substrate device according to one of the preceding claims, wherein
- the first chamber (20) comprises several sub-chambers (20.1, 20.2, 20.3,...) which are arranged adjacent to the second chamber (30), the substrate platform (10) forming a separating wall between the sub-chambers (20.1, 20.2, 20.3,...) and the second chamber (30).

13. Cryopreservation apparatus (200), comprising
- at least one substrate device (100) according to one of the preceding claims, and
- a rotating unit (210), which is adapted for receiving the at least one substrate device (100), wherein
- the substrate device (100), by the rotating unit (210) being capable of pivoting between a cultivation state, in which the substrate platform (10) forms the floor of the first chamber (20), and a temperature control state, in which the substrate platform (10) forms the floor of the second chamber (30).

14. Method for the cryopreservation of a biological sample (1) containing biological cells (2, 2.1, 2.2) with a substrate device (100) or a cryopreservation apparatus (200) according to one of the preceding claims, comprising the steps:
- providing the biological sample (1) on the cultivation surface (11) of the substrate platform (10) in the first chamber (20), the biological cells (2, 2.1, 2.2) being surrounded by a cultivation liquid (3), and
- receiving a cooling medium (5) into the second chamber (30), which is adjacent to the first chamber (20) and is connected to the latter in such a way that the substrate platform (10) forms the separating wall between the first chamber (20) and the second chamber (30), the temperature of the substrate platform (10) being lowered and the biological sample (1) being converted to a frozen state, wherein
the cultivation of the biological cells (2, 2.1, 2.2) comprises the steps:
- providing the first chamber (20) and the second chamber (30) in a cultivation state, in which the substrate platform (10) forms the floor of the first chamber (20) and the cultivation liquid (3) is filled into the first chamber (20), and
- receiving the biological cells (2, 2.1, 2.2) in the adherent state on the cultivation surface (11) of the substrate platform (10), wherein
- the first chamber (20) and the second chamber (30) are pivoted into a temperature control state, in which the substrate platform (10) forms the floor of the second chamber (30), and the cultivation liquid (3) flows out of the first chamber, before the cooling medium (5) is received in the second chamber.

15. Method according to claim 14, wherein the cultivation of the biological cells (2, 2.1, 2.2) comprises the following steps:
- providing the first chamber (20) and the second chamber (30) in a temperature control state, in which the substrate platform (10) forms the floor of the second chamber (30), and
- receiving the biological cells (2) in droplets of the cultivation liquid (3), which are suspended from the cultivation surface (11) of the substrate platform (10).

16. Method according to one of claims 14 and 15, wherein
- the temperature of the substrate platform (10) is lowered at a cooling rate such that the biological sample (1) is vitrified.

17. Method according to one of claims 14 to 16, comprising the step:
- receiving a heating medium (8) into the second chamber (30), the temperature of the substrate platform (10) being raised and the biological sample (1) being converted to a thawed state.

## Revendications

1. Système de substrat (100), qui est mis au point aux fins de la cryoconservation d'un échantillon (1) biologique avec des cellules (2, 2.1, 2.2) biologiques, comprenant
- une plate-forme de substrat (10) pourvue d'un côté avant et d'un côté arrière, dans lequel le côté avant de la plate-forme de substrat (10) forme une surface de culture (11) servant à recevoir l'échantillon (1) biologique,
- une première chambre (20), dans laquelle est contenue la surface de culture (11) de la plate-forme de substrat (10), dans lequel la première chambre (20) est mise au point afin de recevoir un liquide de culture (3), et
- une deuxième chambre (30), qui est mise au point afin de recevoir un milieu de régulation de température (5), dans lequel
- la première chambre (20) et la deuxième chambre (30) sont reliées l'une à l'autre de manière à se jouxter l'une l'autre, et
- la plate-forme de substrat (10) forme une paroi de séparation entre la première chambre (20) et la deuxième chambre (30), dans lequel le côté arrière de la plate-forme de substrat (10) pointe en direction de la deuxième chambre (30),
**caractérisé en ce que**
- le système de substrat (100) comprend une fixation de chambre (50), qui est mise au point pour recevoir de manière pivotante le système de substrat (100) dans un appareil de cryoconservation, dans lequel
- la fixation de chambre (50) comprend deux éléments de support disposés dans un plan de manière parallèle par rapport à l'extension de la plate-forme de substrat (10), lesquels peuvent être couplés à l'appareil de cryoconservation de telle sorte que le système de substrat (100) peut tourner autour d'un axe transversal dans l'appareil de cryoconservation.

2. Système de substrat selon la revendication 1, dans lequel
- le côté arrière de la plate-forme de substrat (10) est dégagé dans la deuxième chambre (30), et
- la plate-forme de substrat (10) est formée avec une épaisseur et une conductivité thermique, qui permettent, lors de l'humidification du côté arrière de la plate-forme de substrat (10) à l'aide d'un premier milieu de régulation de température (5) à une température inférieure à -120 °C, en particulier à l'aide d'azote liquide, une vitrification de l'échantillon (1) biologique au niveau de la surface de culture (11).

3. Système de substrat selon l'une quelconque des revendications précédentes, dans lequel la plate-forme de substrat (10) présente au moins une des propriétés qui comprennent
- la plate-forme de substrat (10) a une épaisseur qui est inférieure à 200 µm, en particulier inférieure à 100 µm,
- la plate-forme de substrat (10) est formée à partir de verre, de matière plastique, d'un matériau semi-conducteur ou de métal, et
- la plate-forme de substrat (10) est formée à partir d'un matériau transparent.

4. Système de substrat selon l'une quelconque des revendications précédentes, dans lequel
- la plate-forme de substrat (10) est reliée de manière amovible à la première ou à la deuxième chambre (20, 30) par l'intermédiaire d'une fixation de substrat (12) étanche aux liquides.

5. Système de substrat selon l'une quelconque des revendications précédentes, qui présente au moins une des caractéristiques qui comprennent
- la plate-forme de substrat (10) est reliée à la première ou à la deuxième chambre (20, 30) par l'intermédiaire d'une section de compensation (13), dans lequel la section de compensation (13) est mise au point afin de recevoir des tensions dépendant de la température entre la plate-forme de substrat (10) et la première ou la deuxième chambre (20, 30), et
- la première chambre (20) présente une soupape de compensation de pression (25), qui est mise au point aux fins de la compensation de pression entre la première chambre (20) et son environnement.

6. Système de substrat selon l'une quelconque des revendications 1 à 4, dans lequel
- la plate-forme de substrat (10) fait partie intégrante de la première ou de la deuxième chambre (20, 30) .

7. Système de substrat selon l'une quelconque des revendications précédentes, qui comprend
- un système d'amenée (40), qui est mis au point afin d'amener le liquide de culture (3) et/ou le milieu de régulation de température (5).

8. Système de substrat selon la revendication 7, dans lequel
- le système d'amenée (40) comprend au moins un élément parmi au moins une conduite de milieu (41, 42) et un système microfluidique (43).

9. Système de substrat selon l'une quelconque des revendications précédentes, dans lequel
- la première et la deuxième chambre (20, 30) sont reliées l'une à l'autre de manière amovible.

10. Système de substrat selon la revendication 9, dans lequel
- la deuxième chambre (30) est reliée de manière solidaire au châssis de chambre (33), qui est mis au point afin de recevoir de manière amovible la première chambre (20).

11. Système de substrat selon la revendication 9 ou 10, dans lequel
- la première chambre (20) est reliée à la deuxième chambre (30) par l'intermédiaire d'un raccord à visser (35).

12. Système de substrat selon l'une quelconque des revendications précédentes, dans lequel
- la première chambre (20) comprend plusieurs chambres partielles (20.1, 20.2, 20.3, ...), qui sont disposées de manière à jouxter la deuxième chambre (30), dans lequel la plate-forme de substrat (10) forme une paroi de séparation entre les chambres partielles (20.1, 20.2, 20.3, ...) et la deuxième chambre (30).

13. Appareil de cryoconservation (200), comprenant
- au moins un système de substrat (100) selon l'une quelconque des revendications précédentes, et
- un système de rotation (210), qui est configuré pour recevoir l'au moins un système de substrat (100), dans lequel
- le système de substrat (100) peut être pivoté avec le système de rotation (210) entre un état de culture, dans lequel la plate-forme de substrat (10) forme un fond de la première chambre (20), et un état de régulation de température, dans lequel la plate-forme de substrat (10) forme un fond de la deuxième chambre (30).

14. Procédé servant à la cryoconservation d'un échantillon (1) biologique avec des cellules (2, 2.1, 2.2) biologiques à l'aide d'un système de substrat (100) ou d'un appareil de cryoconservation (200) selon l'une quelconque des revendications précédentes, comprenant les étapes consistant à
- fournir l'échantillon (1) biologique au niveau de la surface de culture (11) de la plate-forme de substrat (10) dans la première chambre (20), dans lequel les cellules (2, 2.1, 2.2) biologiques sont entourées par un liquide de culture (3), et
- recevoir un milieu de refroidissement (5) dans la deuxième chambre (30), qui jouxte la première chambre (20) et qui est reliée à cette dernière de telle sorte que la plate-forme de substrat (10) forme la paroi de séparation entre la première chambre (20) et la deuxième chambre (30), dans lequel la température de la plate-forme de substrat (10) est baissée et l'échantillon (1) biologique est amené dans un état congelé, dans lequel
la culture des cellules (2, 2.1, 2.2) biologiques comprend les étapes consistant à
- fournir la première chambre (20) et la deuxième chambre (30) dans un état de culture, dans lequel la plate-forme de substrat (10) forme un fond de la première chambre (20) et le liquide de culture (3) est rempli dans la première chambre (20), et
- recevoir les cellules (2, 2.1, 2.2) biologiques dans l'état adhérent sur la surface de culture (11) de la plate-forme de substrat (10), dans lequel
- avant la réception du milieu de refroidissement (5) dans la deuxième chambre, un pivotement de la première chambre (20) et de la deuxième chambre (30) dans un état de régulation de température, dans lequel la plate-forme de substrat (10) forme un fond de la deuxième chambre (30), et un écoulement du liquide de culture (3) sortant de la première chambre ont lieu.

15. Procédé selon la revendication 14, dans lequel la culture des cellules (2, 2.1, 2.3) biologiques comprend les étapes consistant à
- fournir la première chambre (20) et la deuxième chambre (30) dans un état de régulation de température, dans lequel la plate-forme de substrat (10) forme un fond de la deuxième chambre (30), et
- recevoir les cellules (2) biologiques dans des gouttes du liquide de culture (3), qui sont accrochées à la surface de culture (11) de la plate-forme de substrat (10).

16. Procédé selon l'une quelconque des revendications 14 à 15, dans lequel
- la température de la plate-forme de substrat (10) est baissée à un taux de refroidissement tel que l'échantillon (1) biologique est vitrifié.

17. Procédé selon l'une quelconque des revendications 14 à 16, comprenant l'étape consistant à
- recevoir un milieu chauffant (8) dans la deuxième chambre (30), dans lequel la température de la plate-forme de substrat (10) est augmentée et l'échantillon (1) biologique est amené dans un état décongelé.
